# EUROPEAN PATENT APPLICATION

(11) **EP 4 692 117 A1**
(43) Date of publication of application: **11.02.2026**
(21) Application number: 24193824.0
(22) Date of filing: 09.08.2024
(51) Int. Cl.: C07K 16/28, A61K 39/00

(54) **NOVEL OX40 NANOBODIES**

(71) Applicant: Eberhard-Karls-Universität Tübingen, 72074 Tübingen (DE); NMI NATURWISSENSCHAFTLICHES UND MEDIZINISCHES INSTITUT AN DER UNIVERSITÄT TÜBINGEN, 72770 Reutlingen (DE)
(72) Inventor: Rothbauer, Ulrich, 72072 Tübingen (DE); Frecot, Desiree, 72070 Tübingen (DE); Pichler, Bernd, 85298 Fernhag (DE); Kneilling, Manfred, 85298 Scheyern (DE); Sonanini, Dominik, 72072 Tübingen (DE); Maurer, Andreas, 72074 Tübingen (DE); Tränkle, Björn, 72800 Eningen (DE); Kaiser, Philipp, 72076 Tübingen (DE); Wagner, Teresa, 72116 Mössingen (DE)
(74) Representative: Witte, Weller & Partner Patentanwälte mbB

(57) **Abstract**

The present invention relates to novel hOX40-nanobodies, as well as their uses in diagnostic and prognostic appliances, and to methods using the nanobody.

## Description

The present invention relates to novel single-domain-antibodies (sdAb), which are also known as nanobodies, which bind to human OX40 (hOX40/CD134), as well as to the use of such nanobodies, in particular for diagnostic or cell-targeting purposes.

The invention also relates to nucleic acids encoding such nanobodies, to compositions comprising such nanobodies or polypeptides, and to their uses, and method for uses, in particular for diagnostic or cell-targeting purposes.

### BACKGROUND

Immunotherapies that specifically modulate the patient's immune system, e.g., to fight malignant tumor cells or attenuate autoimmune reactions, have opened a new chapter in personalized medicine. Although such therapies have shown remarkable success in some cases, the reasons why patients respond differently need to be understood. Generally, treatment outcomes are highly dependent on the individual immune system and the composition of the tumor (TME) or inflammatory microenvironment (IME).

T cells, a critical component of the adaptive immune system, play a pivotal role in identifying and eliminating cancer cells and managing immune responses. Activated T cells, in particular, are essential in mounting an effective immune response against malignancies and pathogens. The ability to accurately monitor the presence and activity of these cells within various microenvironments is vital for both diagnostic and therapeutic purposes.

In the context of oncology, the tumor microenvironment (TME) is a complex network of cancer cells, stromal cells, immune cells, and extracellular matrix components. The infiltration and activity of activated T cells within the TME are indicative of the immune system's response to the tumor, providing valuable insights into the effectiveness of immunotherapies. Similarly, in immune-mediated diseases, the infiltration of activated T cells within inflamed tissues is a hallmark of disease progression and severity.

Traditional methods for detecting and monitoring activated T cells often lack the specificity, sensitivity, and spatial resolution required for detailed analysis. Therefore, there is a pressing need for advanced tools and probes that can provide real-time, accurate, and non-invasive monitoring of these critical immune cells.

### SUMMARY OF THE INVENTION

This invention addresses this problem by the provision of a nanobody (or "single-domain-antibody"; "Nb"), which specifically binds to human OX40 (hOX40; CD134), wherein the nanobody does not have an agonistic effect on hOX40, the nanobody comprising
a) the amino acid sequences (i) SGFTLDNY (SEQ ID NO: 1) as CDR1, (ii) ISSSGESTNYAD (SEQ ID NO: 2) as CDR2 and (iii) VDDIGTTVQFMCNMGPYEYD (SEQ ID NO: 3) as CDR3; or
b) the amino acid sequences (i) SGFTLEDY (SEQ ID NO: 4) as CDR1, (ii) ISGSGGIRNVAD (SEQ ID NO: 5) as CDR2 and (iii) GFETSYSYSYYCGVHEYD (SEQ ID NO: 6) as CDR3; or
c) the amino acid sequences (i) STDTFSMSA (SEQ ID NO: 7) as CDR1, (ii) ILSDGSTYYAD (SEQ ID NO: 8) as CDR2 and (iii) LRGRLWSNHKDD (SEQ ID NO: 9) as CDR3; or
d) the amino acid sequences (i) SGFTFGSY (SEQ ID No. 13) as CDR1, (ii) IYSDGSTYYAD (SEQ ID No. 14) as CDR2 and (iii) WGAAAPYD (SEQ ID No. 15) as CDR3; or
e) amino acid sequences that have at least 85% sequence homology with the amino acid sequences as defined in a), b), c), or d); or
or a functionally conservative variant of the nanobody as defined in any of a), b), c), or d), comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 13, SEQ ID No. 14, and SEQ ID No. 15.

The novel single domain antibodies of the invention specifically recognize human OX40. With the novel nanobodies disclosed herein, it is possible to selectively identify or target OX40-expressing cells, particularly activated T cells, which makes them a valuable tool in diagnosis, and prophylaxis, alone or in combination/association with additional substances/molecules or methods.

With the new nanobodies, it is possible to distinguish activated T cells from other cell types and detecting subtle changes in their activity and distribution within a respective microenvironment, e.g., within tumor microenvironment (TME), inflammatory microenvironment (IME in immune-mediated diseases (IMIDs) and the lymphatic organs.

Human OX40 (also known as tumor necrosis factor receptor superfamily, member 4 (TNFRSF4), or CD134) is a member of the TNFR-superfamily of receptors which is not constitutively expressed on resting naive T cells. It is expressed about 24 to 72 hours following activation. Accordingly, hOX40 has been described as a surface marker for T cell activation. It is mainly expressed on activated CD8⁺ and CD4⁺ T cells, but also on activated regulatory T cells, natural killer T cells (NKTs) and neutrophils. OX40 binds to the OX40 ligand (OX40L, CD252) presented by activated antigen-presenting cells (APCs) including B cells, dendritic cells and macrophages. OX40-OX40L engagement is key to potentiate T cell responses, including differentiation, proliferation, long-term survival, and enhancement of T cell effector functions, such as cytokine production.

Currently, mouse-specific OX40 monoclonal antibodies (mAbs) are known and applied in imaging studies to predict responses to cancer vaccines in preclinical mouse models, and also to follow the development of acute graft-versus-host disease or rheumatoid arthritis. However, the long systemic half-life of these mAbs (up to seven days after injection) delays prompt imaging and leads to high radiation exposure. Moreover, mAb-based immunoPET has a high background, which limits its sensitivity in detecting small populations of activated T cells.

Consequently, there is a high demand for advanced molecules targeting human OX40 (hOX40) for diagnostic imaging, and the nanobodies presented herein solve this problem. Within the present invention, for the first time, a set of hOX40-specific Nbs is disclosed, which can be used, e.g., to monitor the activation status of human T cells. Further, the inventors of the present invention have demonstrated the capability of nanobodies of the invention for whole-body *in vivo* optical imaging (OI) of hOX40-expressing tumor cells in a mouse xenograft model.

Generally, a single domain antibody (sdAb; or "nanobody" or "Nb") is an antibody fragment consisting of a single monomeric variable antibody domain, which is able to selectively bind to a specific antigen. As single domain antibodies, the nanobodies of the invention represent an excellent alternative to conventional antibodies (IgGs): Antibodies from camelids, such as llamas, include a unique subset of immunoglobulins consisting of heavy chain homodimers devoid of light chains. Their variable region (V_{H}H) is the smallest antigen-binding fragment found in the antibody world, and as a single polypeptide chain it is especially suitable for protein engineering. Single domain antibodies, or "nanobodies", are the recombinant minimal-sized, intact antigen-binding domains derived from the V_{H}H region of these heavy-chain antibodies. Unlike monoclonal antibodies, they can be readily produced in large amounts in simple bacterial expression systems. Moreover, nanobodies are usually extremely stable, can bind antigens with affinities in the nanomolar range, and are smaller in size (approximately 15 kDa) and thereby easier to manipulate genetically as compared with antibody fragments such as single chain variable fragments (scFvs) comprising the variable domains of heavy and light chains of a conventional IgG.

Also, due to the small size and compact folding nanobodies show a high chemical stability, solubility, and fast tissue penetration. Additionally, nanobodies, in particular the ones according to the invention, can be easily converted into multivalent formats, such as biparatopic, bivalent, trivalent, tetravalent or other multi-specific constructs, e.g., addressing different epitopes on the same antigen, which the nanobodies presented herein can also be used for according to the invention. Also, nanobodies have comparable antigen specificities and affinities and, due to their high homology with human antibody (VH) fragments, show only very low immunogenicity.

Accordingly, the nanobodies of the present invention are not antibodies that occur naturally in the human body, but represent synthetically, i.e., biotechnologically generated and engineered antibody fragments.

Throughout the present disclosure, and unless indicated otherwise, the expressions "OX40 nanobody" and "human OX40 nanobody" or "hOX40 nanobody" are interchangeably used and all three expressions designate the same, i.e., a nanobody directed against human OX40, or, in other words, a nanobody binding to human OX40.

The hOX40 nanobodies presented herein, all bind specifically and strongly to human OX40, which has been shown by binding studies against recombinant hOX40 as well as to hOX40 localized at the plasma membrane of mammalian cells.

Also, according to the invention, the nanobody does not have an agonistic effect. Generally, and also within the present invention, an "agonistic effect" means, that the nanobody does not activate the receptor (i.e., hOX40), mimicking the action of the natural ligand (i.e. hOX40L). This means that the nanobody binds to the receptor hOX40 on the surface of an activated T cell and does not induce a biological response similar to what would occur if the receptor's natural activating molecule OX40L (the ligand) were present.

Also, in a preferred embodiment, the hOX40-nanobody of the invention has a level of affinity to OX40, expressed in the dissociation constant (K_{D}), in the pico to low nanomolar ranges, preferably of between including 0.1 nM to including 6 nM, preferably of between including 0.1 nM to including 5 nM, and more preferably of including 0.2 nM to including 3.4 nM. As generally known, the higher the level of affinity of a nanobody/protein to its ligand, the lower the dissociation constant (KD) of the nanobody/ligand-complex. The nanobody of the invention, thus, has a very high level of affinity.

The dissociation constant (KD) can be determined, e.g., by affinity measurements against recombinant hOX40 by biolayer interferometry (BLI), using, e.g., the Octet BLI system (Sartorius, Göttingen, Germany), e.g., the Octet RED96e system, as also outlined in the experimental section.

According to an embodiment, the nanobody of the invention binds to i) domain D1, or to ii) domain D3, of the extracellular region of hOX40. Preferably, the specific binding to the domains can be tested or shown, e.g., by means of using cells expressing the individual domains of human OX40 and by determining the domain specificity of the nanobody by immunofluorescence staining and/or hydrogen deuterium exchange coupled mass spectrometry analysis. In addition, the epitope diversity can be determined via epitope binning analysis and/or hydrogen deuterium exchange coupled mass spectrometry analysis.

Also, in a preferred embodiment, the nanobody of the invention is selected from a nanobody which specifically binds to domain D1 of human OX40, wherein preferably, the nanobody comprises the amino acid sequences a) (i) SGFTLDNY (SEQ ID NO: 1) as CDR1, (ii) ISSSGESTNYAD (SEQ ID NO: 2) as CDR2 and (iii) VDDIGTTVQFMCNMGPYEYD (SEQ ID NO: 3) as CDR3; or b) the amino acid sequences (i) SGFTLEDY (SEQ ID NO: 4) as CDR1, (ii) ISGSGGIRNVAD (SEQ ID NO: 5) as CDR2 and (iii) GFETSYSYSYYCGVHEYD (SEQ ID NO: 6) as CDR3; or amino acid sequences that have at least 85% sequence homology with the amino acid sequences as defined in a), or b); or a functionally conservative variant of the nanobody as defined in any of a) or b), comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6.

The above nanobodies, while binding to the same domain of OX40, i.e. domain 1, preferably bind to at least overlapping epitopes of domain D1 of OX40.

In one embodiment, the nanobody bins to domain D3 of the extracellular region of hOX40, and preferably comprises the amino acid sequences c) (i) STDTFSMSA (SEQ ID NO: 7) as CDR1, (ii) ILSDGSTYYAD (SEQ ID NO: 8) as CDR2 and (iii) LRGRLWSNHKDD (SEQ ID NO: 9) as CDR3; or d) the amino acid sequences (i) SGFTFGSY (SEQ ID No. 13) as CDR1, (ii) IYSDGSTYYAD (SEQ ID No. 14) as CDR2 and (iii) WGAAAPYD (SEQ ID No. 15) as CDR3; or amino acid sequences that have at least 85% sequence homology with the amino acid sequences as defined in c) or d); or a functionally conservative variant of the nanobody as defined in any of c) or d), comprising a conservative substitution of one or two amino acids in one, two or three of the sequences, respectively, SEQ ID No. 7, SEQ ID No. 8, and SEQ ID No. 9; or SEQ ID No. 13, SEQ ID No. 14, and SEQ ID No. 15.

The above nanobodies, while binding to the same domain of OX40, i.e. domain D3, preferably bind to at least overlapping epitopes of domain D3 of OX40.

Also, in a preferred embodiment, the hOX40 nanobody of the invention does not bind to domain D4 of OX40.

OX40 is a 30 kDa glycosylated type 1 transmembrane protein. The extracellular N-terminal portion (Leu29 to Ala216) of OX40 comprises 191 amino acids, and contains four cysteine-rich domains (CRDs). Domain D1 of OX40 comprises 36 amino acids and designated to encompass amino acid positions 29 (Leu) to 65 (Arg), whereas domain D3 comprises 18 amino acids long and designated to encompass amino acid positions 108 (Arg) to 126 (Ala). According to a preferred embodiment, the nanobody of the invention binds to domain D1 of human OX40 between amino acid 29 and amino acid 65; or to domain D3 of human OX40 between amino acid 108 and amino acid 126 and, accordingly, the nanobody of the invention is also a nanobody binding in the respective regions and comprising an amino acid structure as defined herein.

Also, according to a preferred embodiment, the nanobody binds to recombinant and exogenously expressed hOX40, and/or to endogenous hOX40 on activated T cells. Accordingly, in a further preferred embodiment, the nanobody of the invention binds to recombinant and exogenously expressed hOX40 and to endogenous hOX40 on activated T cells. In another further preferred embodiment, the nanobody of the invention binds to recombinant and exogenously expressed hOX40.

In a particularly preferred embodiment, the nanobody of the invention exclusively binds hOX40 on activated T cells.

In a preferred embodiment, the nanobody is an hOX40-OX40L-interaction-blocking hOX40-nanobody, or an inert hOX40 nanobody.

These nanobodies, i.e. the nanobodies of the invention, are useful for diagnostic purposes, e.g., as radio-labeled tracers or fluorescent labeled probes for non-invasive *in vivo* imaging

Presently, and as generally understood, a "hOX40-OX40L-interaction blocking hOX40 nanobody" is a nanobody that does block, *in vivo* and *in vitro,* the binding of OX40 ligand (OX40L, CD252) to hOX40 by binding to hOX40, thus inhibiting the provision of costimulatory signals that support the differentiation, proliferation and long-term survival of T-cells.

In an embodiment of the present invention, the hOX40-OX40L-interaction-blocking hOX40-nanobody binds to domain D1 of hOX40. In a preferred embodiment, the hOX40-OX40L-interaction-blocking hOX40-nanobody is a nanobody comprising/having the amino acid sequences a) (i) SGFTLDNY (SEQ ID NO: 1) as CDR1, (ii) ISSSGESTNYAD (SEQ ID NO: 2) as CDR2 and (iii) VDDIGTTVQFMCNMGPYEYD (SEQ ID NO: 3) as CDR3; or b) the amino acid sequences (i) SGFTLEDY (SEQ ID NO: 4) as CDR1, (ii) ISGSGGIRNVAD (SEQ ID NO: 5) as CDR2 and (iii) GFETSYSYSYYCGVHEYD (SEQ ID NO: 6) as CDR3; or amino acid sequences that have at least 85% sequence homology with the amino acid sequences as defined in a), or b); or a functionally conservative variant of the nanobody as defined in any of a) or b), comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6.

Presently, and as generally understood, an "inert nanobody"/"inert hOX40 nanobody" is a nanobody, that does not block the hOX40-hOX40L-interaction upon binding to hOX40.

In an embodiment of the present invention, the inert hOX40-nanobody binds to domain 3 of hOX40. In a preferred embodiment, the inert hOX40-nanobody is a nanobody comprising/having the amino acid sequences c) (i) STDTFSMSA (SEQ ID NO: 7) as CDR1, (ii) ILSDGSTYYAD (SEQ ID NO: 8) as CDR2 and (iii) LRGRLWSNHKDD (SEQ ID NO: 9) as CDR3; or d) the amino acid sequences (i) SGFTFGSY (SEQ ID No. 13) as CDR1, (ii) IYSDGSTYYAD (SEQ ID No. 14) as CDR2 and (iii) WGAAAPYD (SEQ ID No. 15) as CDR3; or amino acid sequences that have at least 85% sequence homology with the amino acid sequences as defined in c) or d); or a functionally conservative variant of the nanobody as defined in any of c) or d), comprising a conservative substitution of one or two amino acids in one, two or three of the sequences, respectively, SEQ ID No. 7, SEQ ID No. 8, and SEQ ID No. 9; or SEQ ID No. 13, SEQ ID No. 14, and SEQ ID No. 15.

Generally, the amino acid sequence and structure of nanobodies can be considered to include, but are not limited to, four framework regions or "FRs", which are interrupted by three complementarity determining regions or "CDRs". The total number of amino acid residues in a nanobody may be preferably in the range of 110-135.

Thus, according to an embodiment of the present invention, the nanobody comprises four framework regions (FR1 to FR4) and three complementarity determining regions (CDR1 to CDR3), the three complementarity determining regions consisting of
(i) one of the amino acid sequences a), b), c), d), as defined above and in the claims, or of
(ii) an amino acid sequence that has at least 85% sequence homology with one of the amino acid sequences a), b), c), d) or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 13, SEQ ID No. 14, and SEQ ID No. 15;
preferably, wherein the nanobody comprises or consists of an amino acid sequence selected from the group consisting of the amino acid sequences SEQ ID NO. 10, SEQ ID NO. 11, SEQ ID NO. 12, or SEQ ID No. 16; or wherein the nanobody comprises or consists of an amino acid sequence that has at least 85% sequence homology with one of the amino acid sequences SEQ ID NO. 10, SEQ ID NO. 11, SEQ ID No. 12, or SEQ ID No. 16.

In a preferred embodiment of the invention, the nanobody has a sequence homology in the four framework regions (FR1 to FR4) of at least 60%, and a sequence homology in the three complementarity determining regions (CDR1 to CDR3) of at least 85%.

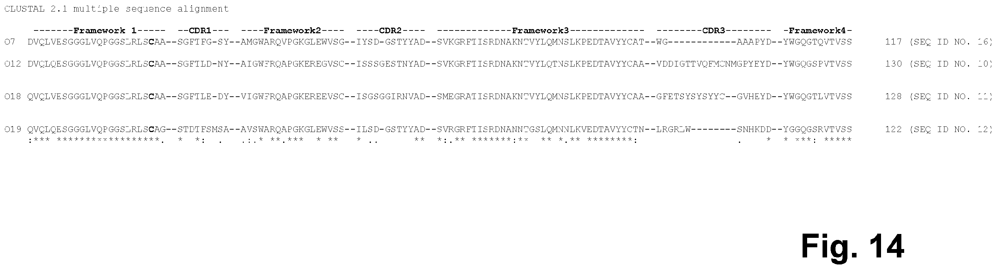

Herein, the amino acid sequences SEQ ID nos. 1 to 9, and 13, 14, and 15 represent CDRs of specific examples for the nanobody/nanobodies of the invention, and the SEQ ID nos. 10, 11, 12 and 16 represent the sequences of the respective full-length nanobody examples, representing specific embodiments of the present invention, and are, respectively, shown in alignment in Fig. 14, and below in table 2.

With exemplarily disclosed nanobodies, the inventors could show that not only an effective, i.e., strong specific binding to activated T cells could be achieved, but also a functionally blocking of the hOX40-hOX40L-interaction could be effected.

Preferably, while the nanobody of the invention strongly and specifically binds to hOX40, it does not increase proliferation of activated OX40-expressing hPBMCs (human peripheral blood mononuclear cells).

The inventors of the present invention could show that exemplary embodiments of the nanobody of the invention did not, or not substantially, increase the proliferation of hPBMCs, in which the hOX40-expression was induced by a stimulation with PHA-L (phytohaemagglutinin L), prior to the exposure to the nanobodies of the invention.

According to a preferred embodiment of the invention, the nanobody is a humanized variant of a nanobody of the invention.

According to another embodiment of the present invention, the hOX40 nanobodies are modified, such, that the binding affinity of the modified nanobodies to hOX40 is substantially retained as compared to the unmodified hOX40 nanobody. Accordingly, with such a modification of the nanobodies of the invention, inert hOX40 nanobodies can be provided, which can be used for, e.g., non-invasive *in vivo* imaging of hOX40 expression. In this regard, a nanobody of the invention, that otherwise has an, e.g., antagonistic OX40-OX40L blocking effect, can be modified, such, that the activated T cell remains non-modulated. This refers, e.g., to the nanobody comprising/having the amino acid sequences a) (i) SGFTLDNY (SEQ ID NO: 1) as CDR1, (ii) ISSSGESTNYAD (SEQ ID NO: 2) as CDR2 and (iii) VDDIGTTVQFMCNMGPYEYD (SEQ ID NO: 3) as CDR3.

The nanobodies of the present invention, as well as the nucleic acids coding for them, i.e., the nucleic acids of the present invention, encompass polypeptides/proteins and nucleic acids having the sequences specified, or sequences substantially identical or similar thereto, e.g., sequences at least 85%, 86%, 87%, 88%, 89% 90%, 91%, 92%, 93%, 94%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences containing a common structural domain having at least about 85%, 86%, 87%, 88%, 89%, 90%, 91%, 92%, 93%, 94%, 95%, 96%, 97%, 98% or 99% identity to a reference sequence. In other embodiments, the amino acid sequence can contain one or more amino acid insertions, deletions, or substitutions (e.g., conservative substitutions) to arrive at a percentage identity of at least about 85%, 86%, 87%, 88%, 89%, 90%, 91 %, 92%, 93%, 94%, 95%, 96%, 97%, 98% or 99% identity to a reference sequence. In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity or encode a common structural polypeptide domain or a common functional polypeptide activity. For example, nucleotide sequences having at least about 80%, 85%, 90%, 91%, 92%, 93%, 94%, 95%, 96%, 97%, 98% or 99% identity to a reference sequence.

Accordingly, a "functionally conservative variant" of the nanobody of the invention as used herein means a nanobody or fragment in which one or more amino acid residues have been modified without altering desired properties, such as antigen affinity and/or antigen specificity. Such variants include, but are not limited to, certain amino acids substituted with amino acids having similar properties. E.g., a "conservative amino acid substitution" is one in which the amino acid residue is replaced with an amino acid residue having a similar side chain. Families of amino acid residues having similar side chains have been defined in the art. Such conservative substituents are preferably substituted by another amino acid residue of the following groups (a) to (e): (a) a small aliphatic, nonpolar or weakly polar residue (Ala, Ser Thr, Pro, Gly), (b) polar, negatively charged residues and their (un-charged) amides (Asp, Asn, Glu, Gln), (c) polar, positively charged residues (His, Arg, Lys) ), (D) large aliphatic, nonpolar residues (Met, Leu, Ile, Val, Cys), and (e) aromatic residues (Phe, Tyr, Trp).

Preferably, an amino acid substitution is performed by means of which the primary amino acid, via which a chemical coupling can take place, is exchanged.

Particularly preferred conservative substitutions are: Ala to Gly or Ser, Arg to Lys, Asn to Gin or His, Asp to Glu, Cys to Ser, Gln to Asn, Glu to Asp, Gly to Ala or Pro, His To Asn or Gin, lie to Leu or Val, Leu to lie or Val, Lys to Arg or Gln or Glu, Met to Leu or Tyr or Ile, Phe to Met or Leu or Tyr, Ser to Thr, Thr to Ser, Trp To Tyr, Tyr to Trp, and / or Phe to Val or lie or Leu, wherein a particularly preferred substitution is Lys to Arg.

In an embodiment of the invention, the nanobody is directly or indirectly associated with at least one detectable label.

With the labeled nanobodies, the applicability of the nanobodies of the invention as labeled "tracer-like" means to visualize and monitor the distribution of hOX40 expressing cells, in particular activated hOX40 expressing T cells, by non-invasive *in vivo* PET (positron emission tomography) could be shown in a tumor microenvironment (TME) and/or inflammatory microenvironment (IME). With the labeled nanobodies of the invention, a strong and stable signal intensity in the tumor organs with increased densities of activated, hOX40 expressing T cells could be shown.

In an embodiment of the present invention, the detectable label is selected from detectable moieties for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

The term "associated with" when used in connection with the nanobody of the invention refers to any modification of the nanobody with another moiety that is/can be used as a diagnostic moiety/agent, or as a therapeutically or pharmacologically active moiety or agent. With the modification, the agent or moiety (the nanobody gets associated with) is indirectly or directly bound to the nanobody or incorporated into the nanobody.

For example, such modification may comprise the introduction (e.g. by conjugation, biologically or chemically linking, covalent binding or in any other suitable manner) of one or more functional groups or agents or moieties, which can be a detectable label, a viral particle, and/or a therapeutically or pharmacologically active agent, and/or that, e.g., alters/increases the half-life, the solubility and/or the absorption of the nanobody, that reduce the immunogenicity and/or the toxicity of the nanobody, that eliminate or attenuate any undesirable side effects of the nanobody, and/or that confer other advantageous properties to and/or reduce the undesired properties of the nanobody; or any combination of two or more of the foregoing. Examples of such functional groups and of techniques for introducing them will be clear to the skilled person and can generally comprise all functional groups and techniques mentioned in the general background art cited hereinabove as well as the functional groups and techniques known *per se* for the modification of pharmaceutical proteins, and in particular for the modification of antibodies or antibody fragments. Such functional groups may for example be linked directly (for example covalently) to a nanobody used in the invention, or optionally via a suitable linker or spacer, as will again be clear to the skilled person.

As used herein the terms "label", "marker" and variants thereof when used in the context of a nanobody/construct refer to a detectable compound, composition or molecule that is linked, associated, or conjugated directly or indirectly to the nanobody or construct disclosed herein, to facilitate detection of the compound. Non-limiting examples of labels as known in the art include fluorescent tags, enzymatic linkages, and radioactive isotopes. In one example, a "labeled nanobody" refers to the direct or indirect conjugation of the detectable compound, composition, or molecule to the nanobody. Additionally, or alternatively, the detectable compound, composition or molecule can be incorporated into the nanobody structure by means other than direct or indirect conjugation. An exemplary method of such incorporation is the replacement of an amino acid residue of the nanobody with a modified amino acid residue such that it becomes detectable (e.g., by radiolabeling). The label may be directly detectable or may be detectable only after contact with further compounds compositions or molecules. In one, non-limiting example, the label may be the incorporation of a radiolabeled amino acid, which is directly detectable according to methods known in the art. In additional or alternative non-limiting examples, the label may be the attachment of biotinyl moieties to the nanobody, which are detectable following contact with marked avidin (for example, streptavidin containing a fluorescent marker or enzymatic activity that can be detected by optical or colorimetric methods as known in the art). Various methods of labeling proteins are known in the art and may be used.

In an embodiment of the invention, in particular where the nanobody of the invention is used in diagnostic imaging methods, the nanobody is associated with a functional entity via which a detectable entity can be attached to the nanobody. E.g., the nanobody of the invention is combined with a chelator, and via the chelator, e.g., a radionuclide is attached to the nanobody. The chelator can be any "bifunctional chelating agent" having the capability to chelate (radio-)metal ions and to covalently bind the nanobody/nanobodies of the invention. Exemplary bifunctional chelating agents include, but are not limited to, DTPA, NOTA, benzyl-NOTA, alkyl or aryl derivatives of NOTA, NODA, NODA-GA, C-NETA, succinyl-C-NETA and bis-t-butyl-NODA. As mentioned, via the chelator, e.g., a radionuclide can be attached to/associated with the nanobody of the invention, in particular with the radionuclides as discussed below.

According to an embodiment of the invention, examples for detectable labels for polypeptides/proteins include, but are not limited to, the following: radioisotopes or radionuclides (such as ³⁵S, ¹¹C, ¹³N, ¹⁵O, ¹⁸F, ¹⁹F, ⁶⁴Cu, ⁹⁹TC, ¹³¹I, ³H, ¹⁴C, ¹⁵N, ⁸⁹Zr, ⁹⁰Y, ⁹⁹Tc, ¹¹¹In and ¹²⁵I), fluorescent labels (see below), enzymatic labels (such as horseradish peroxidase, beta-galactosidase, luciferase, alkaline phosphatase), chemiluminescent markers, biotinyl groups, predetermined polypeptide epitopes recognized by a secondary reporter (such as a leucine zipper pair sequences, binding sites for secondary antibodies, metal binding domains, epitope tags), or magnetic agents (such as gadolinium chelates). In some embodiments, labels are attached by spacer arms of various lengths to reduce potential steric hindrance for the binding of the nanobody or the construct disclosed herein.

Also, by "fluorescent label" mentioned above, any molecule is meant that may be detected via its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, CASCADE BLUE^{®}, TEXAS REDO, IAEDANS, EDANS, BODIPY FL, LC Red 640, Cy 5, Cy 5.5, LC Red 705 and OREGON GREEN^{™}. Suitable optical dyes are described in the 1996 Molecular Probes Handbook by Richard P. Haugland, hereby expressly incorporated by reference. Suitable fluorescent labels also include, but are not limited to, green fluorescent protein (GFP), blue fluorescent protein (BFP), enhanced yellow fluorescent protein (EYFP).

Particularly preferred labels for use in the present invention include: ALEXA FLUOR^{®} dyes (sulfonated amino-coumarin or rhodamine) (ALEXA FLUOR^{®} 350, ALEXA FLUOR^{®} 430, ALEXA FLUOR^{®} 488, ALEXA FLUOR^{®} 546, ALEXA FLUOR^{®} 568, ALEXA FLUOR^{®} 594, ALEXA FLUOR^{®} 633, ALEXA FLUOR^{®} 647, ALEXA FLUOR^{®} 660, ALEXA FLUOR^{®} 680), CASCADE BLUE^{®} (pyrenyloxytrisulfonic acid), CASCADE YELLOW^{™} and R-phycoerythrin (PE), FITC, Rhodamine, and TEXAS REDO (tetramethyirhodamines with extra julolidine rings).

To "label" a nanobody of the invention, any method known in the field can be applied. Generally, to label the nanobody of the invention for, e.g., positron emission tomography (PET), PET tracers are coupled to a chelator (e.g., NODAGA) or click-reagent via a reactive unit (e.g., primary amino group, azide group in combination with DBCO, C-terminal cysteine, His6-tag) located within the nanobody, via which the radionuclide is then introduced.

According to an embodiment, the nanobody of the invention may be labelled, e.g., via a chelator as discussed above, with an radio-isotope chosen from the group consisting of Actinium-225, Astatine-211 , Bismuth-212, Bismuth-213, Caesium-137, Chromium-51 , Cobalt-60, Dysprosium -165, Erbium-169, Fermium-255, Gold-198, Holium-166, lodine-125, lodine-131 , Iridium-192, Iron-59, Lead-212, Lutetium-177, Molydenum-99, Palladium-103, Phosphorus-32, Potassium-42, Rhenium-186, Rhenium-188, Samarium-153, Technitium-99m, Radium-223 , Ruthenium-106, Sodium-24, Strontium-89, Terbium-149, Thorium-227, Xenon-133, Ytterbium-169, Ytterbium-177, Yttrium-90.

According to one aspect of the invention, the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

With the nanobody of the invention modified with a detectable label or "a contrast agent", different approaches for imaging cells can be performed. E.g., non-invasive imaging approaches offer a significant benefit compared to current diagnostic standard, since, currently, e.g., suitable rodent models for *in vivo* application of hOX40 nanobodies are not available.

Accordingly, in an embodiment of the nanobody of the invention, the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

The present invention also relates to a nucleic acid comprising or consisting of a nucleic acid sequence coding for the nanobody as defined above, optionally linked to another nucleic sequence.

Accordingly, the nucleic acid of the invention is a nucleic acid encoding an amino acid sequence comprising framework region 1, CDR1, framework region 2, CDR2, framework region 3, CDR3 and framework region 4, in this order, of the nanobody as described herein, as well as a polypeptide comprising at least one nanobody as described herein.

In some of the aspects described herein, a nucleic acid sequence encoding a nanobody of the invention, or any module thereof, is operably linked to a vector. In general, as used herein, the term "vector" refers to any genetic element, such as a plasmid, phage, transposon, cosmid, chromosome, virus, virion, etc., that is capable of replication when associated with the proper control elements and that can transfer gene sequences to cells. Thus, the term includes cloning and expression vehicles, as well as viral vectors. By "recombinant vector" is meant a vector that includes a heterologous nucleic acid sequence, or "transgene", that is capable of expression *in vivo.* Vectors useful for the delivery of a sequence encoding a nanobody of the invention or components thereof can include one or more regulatory elements (e.g., promoter, enhancer, etc.) sufficient for expression of the nanobody or component thereof in the desired target cell or tissue.

In an embodiment, the nanobody of the invention, or the nucleic acid of the invention, is/are used, as already addressed above, in diagnostic or prognostic methods, preferably imaging methods in or for a patient.

By "diagnostic method" or "diagnosis", is meant here a method giving the possibility of determining whether in an individual, or a sample of an individual, in particular in, e.g., within tumor microenvironment (TME), inflammatory microenvironment (IME in immune-mediated diseases (IMIDs) and the lymphatic organs, activated OX40 expressing T cells are present, absent, or are present in certain amount. Via this assessment, it is possible to monitor OX40 expression in immune diagnostics.

By "prognostic method" or "prognosis", is meant here a method giving the possibility of determining, e.g., whether an individual risks developing a status or pathology.

The patient or individual, as discussed in and throughout the present invention, is preferably a mammal, in particular a mammal selected from a human or an animal mammal, the latter preferably being selected from non-human primates, rodents, in particular rats, mice.

Preferably, in an embodiment of the invention, the nanobody of is used as contrast agent in non-invasive medical imaging *in vivo,* preferably for identifying the presence, absence, and/or amount of hOX40-expressing cells, preferably of hOX40-exrepssing T cells, preferably in a patient or a sample of a patient.

In an embodiment of the invention, the nanobody is used for monitoring of hOX40 expression in cancer diagnostics, diagnostics of immune mediated diseases, and/or evaluation of immunotherapy.

The diseases or conditions that may be monitored using the nanobody of the invention are selected from cancer, autoimmune diseases (immune mediated diseases), colitis, allogenic stem cells transplantation, organ transplant rejection, acquired immunodeficiency disease, and also include the ones listed below. The immune-mediated or "autoimmune" disease may be at least one selected from the group consisting of, but not limited to, rheumatoid arthritis, type 1 diabetes, systemic scleroderma, systemic lupus erythematosus, atopic dermatitis, psoriasis, alopecia areata, asthma, Crohn's disease, Behcet's disease, chronic thyroiditis, multiple sclerosis, polymyositis, ankylosing spondylitis, fibromyositis, and polyarteritis.

Non-limiting examples of cancers or neoplastic conditions, the status of which (e.g., during or after a cancer treatment of a patient) can be monitored with the nanobody of the invention, include a fibrosarcoma, myosarcoma, liposarcoma, chondro-sarcoma, osteogenic sarcoma, gastric cancer, esophageal cancer, rectal cancer, pancreatic cancer, ovarian cancer, prostate cancer, uterine cancer, cancer of the head and neck, skin cancer, brain cancer, squamous cell carcinoma, medullary carcinoma, bronchogenic carcinoma, renal cell carcinoma, hepatoma, bile duct carcinoma, cervical cancer, testicular cancer, small cell lung carcinoma, non-small cell lung carcinoma, bladder carcinoma, epithelial carcinoma, glioma, astrocytoma, medulloblastoma, melanoma, neuroblastoma, retinoblastoma, leukemia, lymphoma, or Kaposi sarcoma.

By "solid tumor(s)" or "tumor(s)" are meant primary tumors and/or metastases (wherever located) such as but not limited to gliomas, pancreatic tumors; lung cancer, e.g. small cell lung cancer, breast cancer; epidermoid carcinomas; neuroendocrine tumors; gynecological and urological cancer, e.g. cervical, uterine, ovarian, prostate, renal-cell carcinomas, testicular germ cell tumors or cancer; pancreas cancer (pancreatic adenocarcinoma); glioblastomas; head and/or neck cancer; CNS (central nervous system) cancer; bones tumors; solid pediatric tumors; hematological malignancies; AIDS-related cancer; soft-tissue sarcomas, and skin cancer, including melanoma and Kaposi's sarcoma.

The present invention also concerns a multiparatopic, biparatopic, bivalent or trivalent construct comprising at least one nanobody of the invention or a combination of several nanobodies of the invention.

A "multiparatopic construct" as used herein and as generally understood refers to a polypeptide construct that binds to two or more different epitopes on one antigen, e.g., two (= biparatopic) or three (= triparatopic). Accordingly, the present invention also comprises constructs comprising the binding sequences of at least two of the nanobodies of the invention.

A "multivalent construct", on the other hand, is a construct that has at least two antigen binding sites/binding units. In some embodiments, a multivalent construct is a bivalent construct, trivalent construct, or a tetravalent construct.

Within the present invention, "at least one nanobody" includes "only one nanobody" of the invention, but also more than one nanobodies of the invention, e.g., 2, 3, 4, 5, 6 or 7 different nanobodies of the invention.

The present invention also concerns a method for determining and/or imaging and/or monitoring the distribution, presence, absence, and/or amount of hOX40-expressing cells in a biological sample or tissue, the method comprising: contacting the biological sample or tissue with at least one nanobody of the invention and determining and/or imaging and/or monitoring the distribution, presence, absence, and/or amount of hOX40-expressing cells in the biological sample or tissue. Preferably, the biological sample and tissue is from/in a human patient. The tissue is preferably an organ of a human patient.

In particular, in a preferred embodiment of the invention, the method comprises the steps of
a) contacting said biological sample or tissue with at least one of the nanobodies of the present invention, wherein said nanobody is associated with a detectable label as outlined above,
b) measuring, via the detectable label, imaging signals from said nanobody that has bound to hOX40-expressing cells in said biological sample or tissue, and
c) imaging, determining and/or monitoring the presence, absence, and/or amount and/or activity of OX40-expressing cells, in said biological sample or tissue via the imaging signals,
thereby determining and/or monitoring the distribution of OX40-expressing cells in the biological sample or tissue.

The biological sample can be any sample or a portion of a larger biological element. Preferably, the sample is a substance of biological origin. Examples of biological samples include, but are not limited thereto, portions of organs or tissues such as the kidney, the liver, heart, the lung, etc., the arteries, the veins, etc., the blood, e.g. whole blood, and its compounds such as the plasma, serum, the platelets, the sub-populations of blood cells etc., as well as saliva, sputum, or any sample retrieved from the mouth and pharynx region, also e.g. by using a mouth wash or rinse containing saliva or sputum samples. The biological sample, preferably, is from a mammal, preferably a human. Also, preferably, the biological sample can be a cell culture comprising cells derived from a mammal, preferably a human, such as primary cells or (established) cell lines, with a "primary cell culture" being the culture of cells directly isolated from a tissue of interest and retaining the morphological and functional characteristics of their tissue of origin. Whereas cell lines are cells that have been continually passaged over a long period of time and have acquired homogenous genotypic and phenotypic characteristics; they can be finite or continuous.

The tissue can be any tissue of a mammal, preferably a human, i.e., the method can be performed on living mammals, e.g., a human, so that non-invasive *in vivo* imaging can be performed. By applying this method, e.g., tumor growth or size can be determined and monitored.

In an embodiment of the method of the invention, the nanobody is detectably labeled, and in the determining and/or imaging and/or monitoring step the presence, or absence, or distribution, or amount of the label is detected.

Accordingly, the present invention also relates to the use of at least one of the nanobodies of the present invention, or of at least one construct of the present invention, as a diagnostic agent, preferably wherein the diagnostic agent is used in a diagnosis which is selected from diagnostic classification of a disease-associated status of a patient, susceptibility to a medical therapy, such as an immunotherapy, of a patient.

In another embodiment of the method of the invention, the at least one nanobody of the present invention, or at least one construct of the present invention, is used to assess and/or monitor hOX40-expressing cells in a biological sample, preferably of a patient, wherein the nanobody is used alone, or in combination with, or sequentially relative to at least one other, i.e., second, third, fourth, fifth, sixth, seventh, eight, nineth, tenth, etc., cell-specific marker. The at least one other cell-specific marker can be any marker of interest and suitable for detecting a cell/cells of interest expressing hOX40 and the second cell-specific marker.

Accordingly, in an embodiment, the method comprises the steps of
a) combining said sample with at least one of the nanobodies of the invention, or with at least one construct of the invention, and with at least one second compound conjugated with a detectable label, the second compound binding to another marker of the hOX40-expressing cells, preferably a T cell marker,
b) measuring, via the detectable label, imaging signals from said nanobody that has bound to hOX40 expressing cells in said sample, and, if a second compound has additionally been used in step a), from said second compound that has bound to another marker; and
c) imaging, determining and/or monitoring the presence and/or amount and/or activity of hOX40 expressing cells and the presence and/or amount of the second marker, preferably T cell marker, in said sample via the imaging signals.

In an embodiment of the combined use, the second cell-specific marker is a compound, e.g. an antibody/nanobody or fragment thereof, binding to a T-cell marker wherein the T cell-specific marker is selected from at least one of CD2, CD3, CD7, CD8, CD27, CD28, CD127, HLA-DR (MHC II), CD38, CD69, CCR4, CCR5, CCR6, CCR7, CTLA-4, LAG3, TIM-3, ICOS, CXCR4, CXCR1, CXCR2, PD-1, PD-L1, PD-L2, CD40L (synonym CD154), CD122, CD137, GITR, CD25, CD278, SIGLEC-7, SIGLEC-9, BTLA (synonym: CD272), TIGIT, VISTA, B7-H4 (synonym: VTCN1), CD276 (synonym: B7-H3), A2AR, CEACAM1, LAIR-3, HVEM, CD160, CD200, CD200R, CD206 (MMR), CD163, CD11a, CD11b, CD11c, CD14, CD86, CD34, CD45, CD80, IL-1R, IL-4 R alpha, TLR1, TLR2, TLR4, CD31, CD68, CD15, Fc gamma RI (CD64), Fc gamma RII (CD32), Fc gamma RIII (CD16), EMR1, galectin-3, M-CSF R/CD115, Siglec-3/CD33, CD36, INF gamma R, CD209, CD150.

In an embodiment of the method of the invention, the nanobody and the second compound is detectably labeled, preferably radiolabeled, and wherein in the determining and/or imaging and/or monitoring step the presence, or absence, or distribution, or amount of the label is detected.

The present invention also relates to a cell line producing a nanobody of the invention.

Also, the present invention relates to the use of at least one of the nanobodies of the invention, or of at least one construct of the invention, as a diagnostic agent, preferably wherein the diagnostic agent is used in a diagnosis which is selected from diagnostic classification of a disease-associated immune status of a patient, and susceptibility to immunotherapies of a patient.

Also, the present invention relates to a kit comprising at least one nanobody of the invention, the nucleic acid coding for the nanobody of the invention, or a cell of a cell line of the invention; and a detectable marker.

Also, it is noted that by providing the amino acid sequence of the nanobody of the invention, one skilled in the art is capable of producing the nanobodies according to the invention and described herein, by conventional techniques for producing polypeptides. For example, they may be synthesized by using a well-known synthesis method in a solid phase.

Alternatively, the nanobodies according to the invention may be synthesized with recombinant DNA techniques well known one skilled in the art (Maniatis *et al.* (1982) Molecular Cloning: a laboratory manual, Cold Spring Harbor Laboratories, NY, 51-54 and 412-430). For example, they may be obtained as DNA expression products after incorporating DNA sequences coding for the polypeptide of interest in expression vectors and introducing these vectors in suitable prokaryotic or eukaryotic hosts which will express the polypeptide of interest, from which they may then be isolated by using techniques well known to one skilled in the art. Accordingly, the invention also concerns a vector comprising a nucleic acid coding for the polypeptides and nanobodies as disclosed herein, as well as a transfected, infected or transformed cell with such a nucleic acid or vector.

Further advantages follow from the description of the embodiments and the attached drawings. All publications, patent applications, patents, and other references mentioned herein are incorporated by reference in their entirety.

Further, it is noted that, as used herein, the articles "a" and "an" refer to exactly (or only) one or to more than one (e.g., to at least one) of the grammatical object of the article.

The term "or" is used herein to mean, and is used interchangeably with, the term "and/or", unless context clearly indicates otherwise.

The terms "proteins" and "polypeptides" are used interchangeably herein, as well as the terms "nanobody" and "single domain antibody".

Also, the terms "about" and "approximately" and "substantially" shall generally mean an acceptable degree of error for the quantity measured given the nature or precision of the measurements. Exemplary degrees of error are within 20 percent (%), typically, within 10%, and more typically, within 5% of a given value or range of values.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description.

### BRIEF DESCRIPTION OF THE DRAWINGS

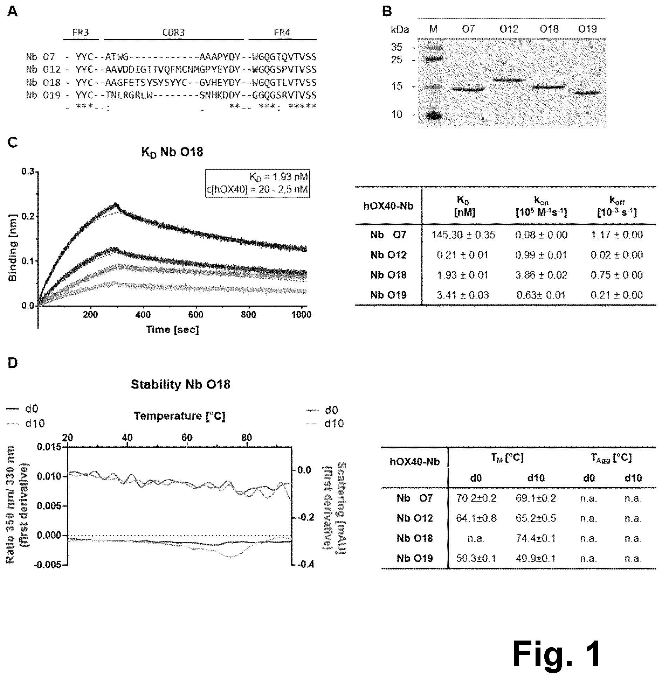

**Fig. 1** shows the results of the biochemical characterization of hOX40-Nbs: (**A**) Amino acid (aa) sequences of the complementarity determining region (CDR) 3 from 4 unique hOX40 Nbs identified by two consecutive rounds of bio panning (full sequences are displayed in Table 2 below). (**B**) Coomassie-stained SDS-PAGE of 2 µg purified hOX40-Nbs after purification using immobilized metal affinity chromatography (IMAC) and size exclusion chromatography (SEC). (**C**) Biolayer interferometry (BLI)-based affinity measurements exemplarily shown for Nb 018. Biotinylated Nb was immobilized on streptavidin biosensors. Kinetic measurements were performed using four concentrations of recombinant hOX40 ranging from 2.5 nM - 20 nM (displayed with gradually lighter shades of color/grey; left). Summary table (right) shows affinities (KD), association constants (kon), and dissociation constants (koff) determined by BLI using four concentrations of purified Nbs as mean ± SD. (**D**) Stability analysis using nano scale differential scanning fluorimetry (nanoDSF) displaying fluorescence ratio (350 nm/330 nm) (red) and light scattering (gray) shown as first derivative for day 0 (dark shade) and after an accelerated aging period of 10 days at 37°C (light shade), exemplary shown for Nb 018 (left) and summarized for all hOX40-Nbs in the table (right). Data are shown as mean value of three technical replicates;

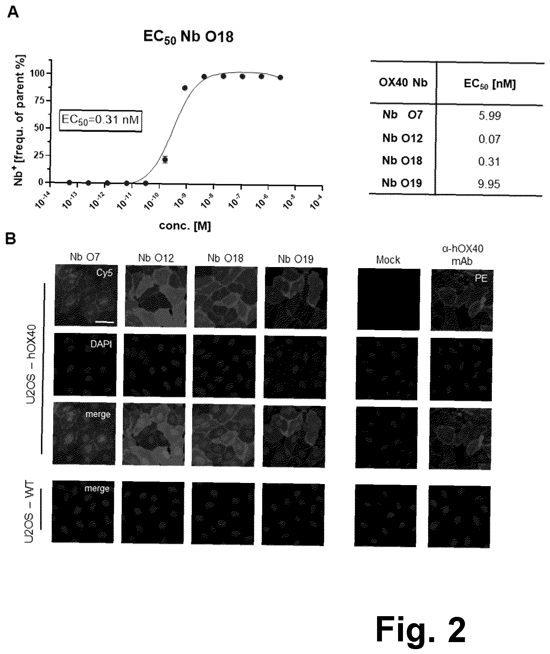

**Fig. 2** shows the results of experiments for the characterization of cellular binding of nOX40-Nbs: (**A**) Determination of hOX40-Nb binding to cellular expressed hOX40 by flow cytometry (n=3), exemplary shown for Nb 018 labeled with AlexaFluor647 (AF647; left). The percentage of positively stained U2OS-hOX40 (frequency of parent) was plotted against indicated concentrations of AF647-labeled hOX40-Nbs and EC50 values shown in table (right) were calculated from a four-parametric sigmoidal model based on the mean ± SD of three technical replicates. (**B**) Representative images of U2OS-hOX40 cells (upper panel) and U2OS-WT cells (lower panel) stained with AF647-labeled hOX40-Nbs (left) as well as an unspecific Nb (Mock) as negative and phycoerythrin (PE)-labeled OX40 mAb as positive control (right). Shown are individual Nb staining, nuclei staining (Hoechst, blue) and merged signals; scale bar: 50 µm;

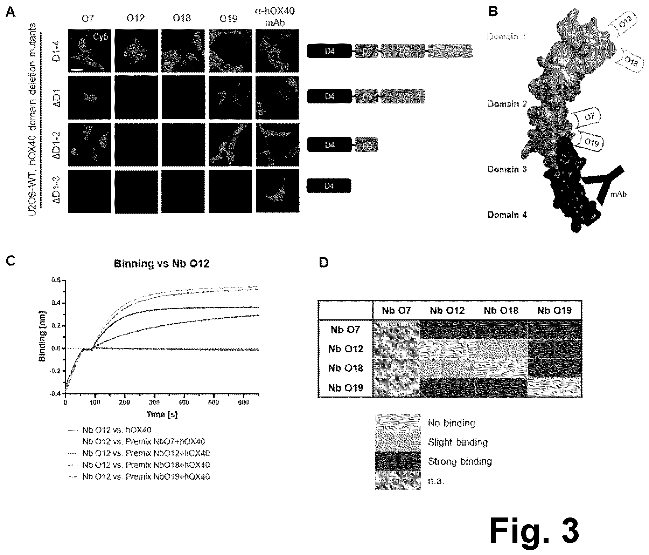

**Fig. 3** shows the results of experiments for the characterization of binding epitopes of hOX40-Nbs: (**A**) Domain mapping by immunofluorescence staining with hOX40-Nbs on U2OS cells displaying either surface exposed hOX40 full length (D1-4), or domain deletion mutants as indicated. Shown are representative images of living cells stained with individual AF647-labeled Nbs or anti-OX40 Ab; scale bar: 50 µm. (**B**) Schematic overview summarizing the results of domain mapping analysis (crystal structure OX40 PDB: 2HEV). (C) Epitope binning analysis of hOX40-Nbs by BLI. Representative sensograms of combinatorial Nb binding to recombinant hOX40 on sharing/overlapping epitopes or on different epitopes are shown (left). (D) Graphical summary of epitope binning analysis;

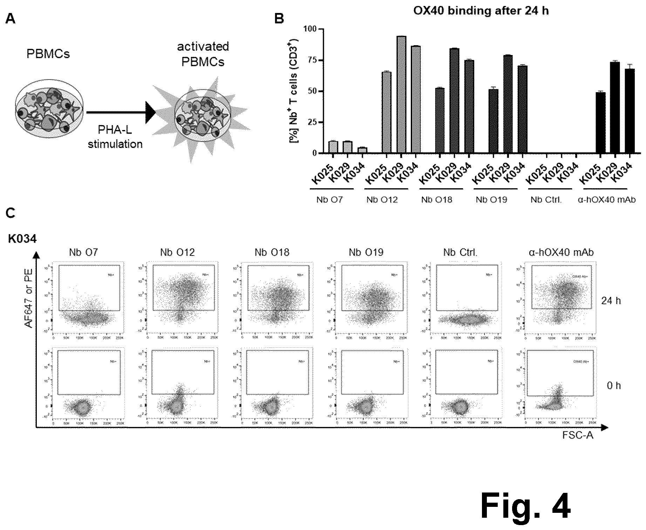

**Fig. 4** shows the results of experiments for validating of hOX40-Nb binding to activated T cells: (**A**) Schematic outline of activation of human peripheral blood mononuclear cells (hPBMCs) by phytohaemagglutinin L (PHA-L) stimulation. (**B**) Flow cytometry analysis of hOX40-Nbs staining on CD3⁺ hPBMCs from three different donors (K025, K029 and K034) after 24 h of PHA-L stimulation shown as bar graph. Data are presented as mean ± SD of three technical replicates. (**C**) Exemplary results of flow cytometry analysis of CD3⁺ hPBMCs derived from donor K034 stained with AF647-labeled hOX40-Nbs, an unspecific Nb (Nb. Ctrl.) or an PE-labeled hOX40 mAb before (0 h, lower panel) and after (24 h, upper panel) PHA-L stimulation;

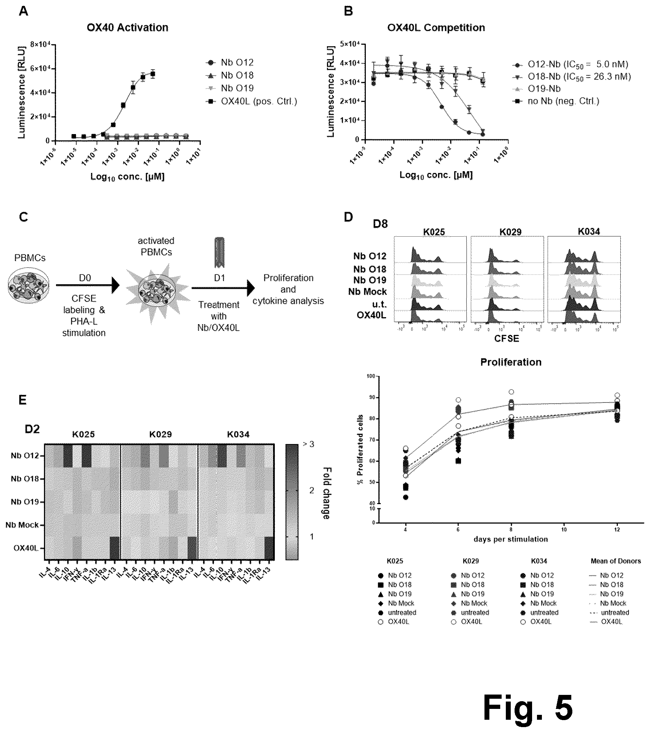

**Fig. 5** shows the results of experiments for the analysis of hOX40-Nbs on OX40-signaling: (**A**, **B**) Assessment of agonistic or antagonistic activities of hOX40-Nbs on OX40 signaling in a cell-based OX40 bioassay. (**A**) For determining agonistic effects OX40 effector cells were treated for 5 h with a serial titration of Nbs 012, 018, 019 or OX40L as positive control (pos. Ctrl.) followed by luminescence detection using Bio-Glo reagent. Data are shown as a three-parameter logistic regression dose-response curve based on the mean ± SD of three technical replicates (n = 3). (**B**) For analysis a potential OX40L competition, OX40 effector cells were preincubated with serial dilutions of Nbs ranging from 0.13 µM to 0.002 nM before adding OX40L at the saturation concentration of 0.12 µM followed by luminescence detection using Bio-Glo reagent. Three-parameter logistic regression dose-response curves based on the mean ± SD of three technical replicates showed an antagonistic effect of Nb 012 and 018 with IC₅₀ values of ~ 5.0 nM or 26.3 nM, respectively. (**C**) Schematic workflow for testing the impact of Nbs on T cell proliferation and cytokine release. hPBMCs of three donors (K025, K029, K034) were CFSE-labeled and stimulated with 5 µg/mL PHA-L. After 24 h, hPBMCs were treated with 0.5 µM hOX40-Nbs, unspecific Nb (Mock), OX40L or left untreated (u.t.). Proliferation at days 4, 6, 8 and 12 and cytokine release at day 2, 4 and 8 were monitored. (**D**) Proliferation was analyzed by flow cytometry (CFSE-low/negative fraction), exemplary shown for day 8 (D8, upper panel). Mean percentages of all three donors are shown as plain or dotted lines (lower panel). (**E**) Determination of cytokines secreted after treatment with hOX40-Nbs displayed as a heat map, exemplary shown for day 2 (D2) after treatment. Values are shown as fold change compared to the untreated control based on the mean of three technical replicates;

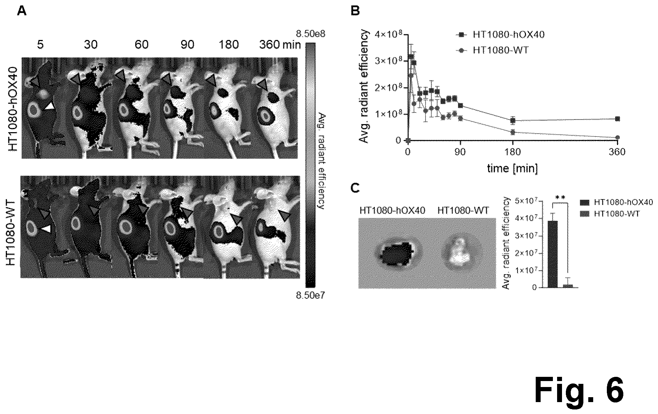

**Fig. 6** shows the results of experiments of *in vivo* optical imaging with Nb O18_{AF647}: *In vivo* optical imaging (OI) with O18_{AF647} in HT1080-hOX40 and HT1080-WT tumor bearing mice. 5 µg of O18_{AF647} were administered intravenously (i.v.) to CD1 nude mice subcutaneously injected with human HT1080-hOX40 or HT1080-WT at the right upper flank. Tumor biodistribution was monitored by repetitive OI measurements over the course of 6 h (**A**) Acquired images of different measurement time points of one representative O18_{AF647} injected mouse with a HT1080-hOX40 (top) or HT1080-WT (bottom, control). Red arrows indicate the tumor localization at the right upper flank. The kidney is marked with a white arrow at the 5 min time point. (**B**) Quantification of the fluorescence signal from the tumors (n = 3 per group, arithmetic mean of the average radiant efficiency ± SEM) determined at indicated time points. (**C**) Representative *ex vivo* OI of harvested tumor (left) and organ quantification of O18_{AF647} in HT1080-hOX40 and HT1080-WT tumors (n = 3 per group, arithmetic mean ± SEM; unpaired t test, p = 0.0031);

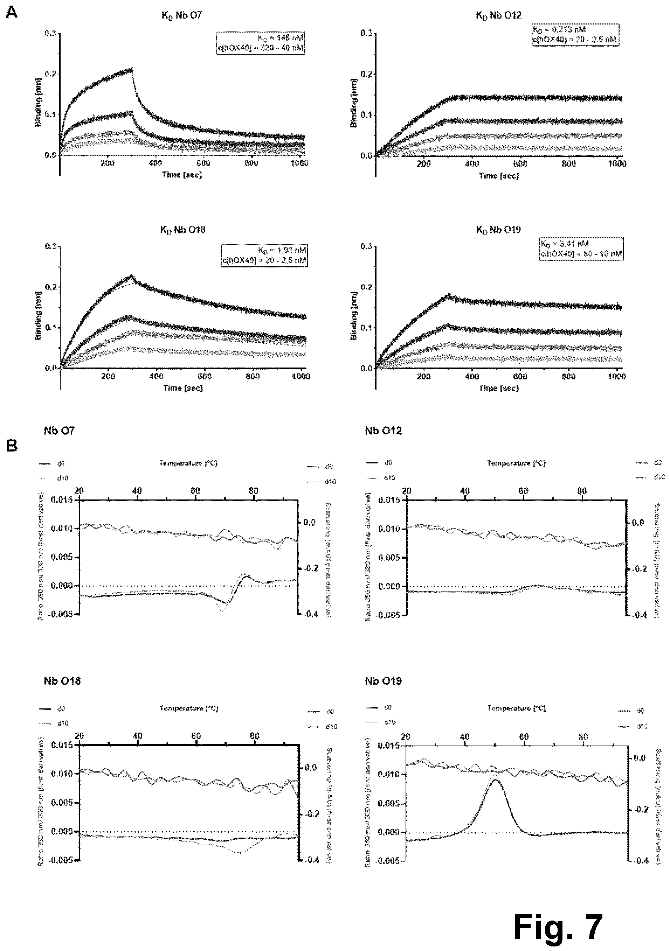

**Fig. 7** shows the results of experiments on affinity and stability measurements of hOX40-Nbs: (**A**) Biolayer interferometry (BLI)-based affinity measurements were performed by immobilization of biotinylated hOX40-Nbs on streptavidin sensors. Kinetic measurements were performed using four concentrations (as indicated) of recombinant hOX40 (displayed with gradually lighter shades of color/grey). The indicated binding affinities (K_{D}) were calculated from global 1:1 fits shown as dashed lines. (**B**) Stability analysis of individual hOX40-Nbs using nano scale differential scanning fluorimetry (nanoDSF) displaying fluorescence ratio (350 nm/330 nm) (red) and light scattering (gray) are shown as first derivatives for day 0 (dark shade) and after an accelerated aging period of 10 days at 37°C (light shade);

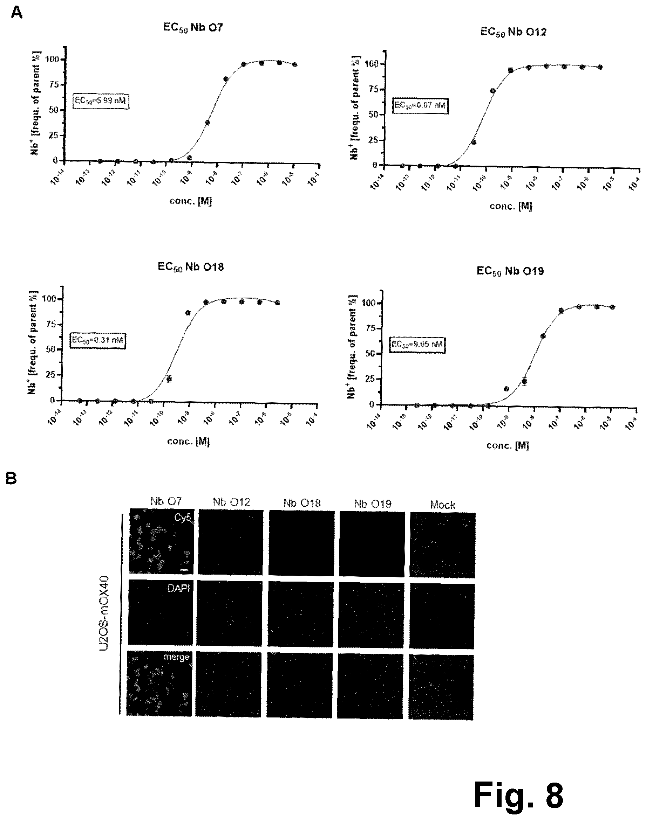

**Fig. 8** shows the results of experiments for characterization of cellular binding of hOX40-Nbs: (**A**) Determination of hOX40-Nb binding to cellular expressed hOX40 by flow cytometry. The percentage of positively stained U2OS-hOX40 cells with AlexFluor647- (AF647) labeled hOX40-Nb (frequency of parent) was plotted against indicated concentrations of hOX40-Nbs and indicated EC₅₀ values were calculated from a four-parametric sigmoidal model based on the mean ± SD of three technical replicates (n = 3). (**B**) Cross-reactivity analysis of hOX40-Nbs by immunofluorescence staining. Representative images of U2OS cells transiently expressing murine OX40 (U2OS-mOX40) cells stained with AF647-labeled hOX40-Nbs or an unspecific Nb (Mock) as negative control. Shown are staining with individual Nbs, nuclei staining (Hoechst, blue) and merged signals; scale bar: 50 µm;

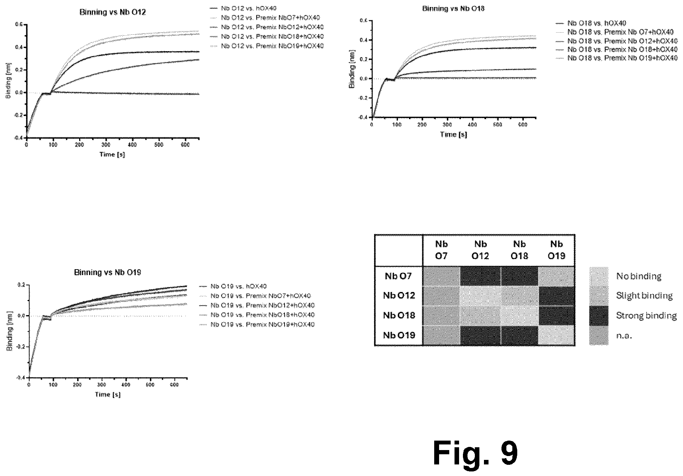

**Fig. 9** shows the results of epitope binning experiments of hOX40-Nbs by biolayer interferometry (BLI): Biotinylated first hOX40-Nb was immobilized on streptavidin biosensors. hOX40 (100 nM) was pre-incubated (premix) in 10-fold excess with designated second hOX40-Nb (as indicated) followed by determining additional association of the hOX40/Nb complex to the immobilized first hOX40-Nb. All sensograms of combinatorial Nb binding to hOX40 on sharing/overlapping epitopes or on different epitopes including a graphical summary of epitope binning analysis are shown;

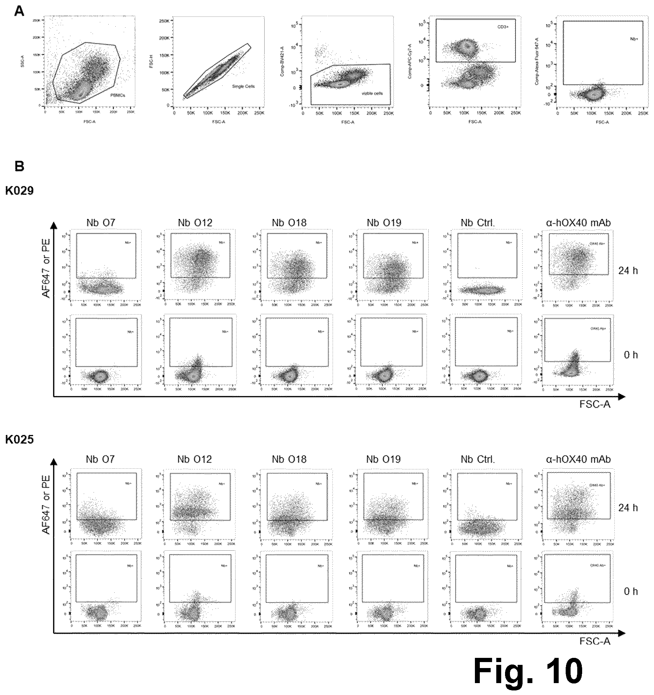

**Fig. 10** shows the results of experiments for validation of hOX40-Nb binding to activated T cells: (**A**) Gating strategy for flow cytometry analysis of hOX40-Nb staining of CD3⁺ cells derived from human PBMCs (hPBMC). (**B**) Flow cytometry analysis of CD3⁺ hPBMCs derived from donors K029 and K025 stained with AF647-labeled hOX40-Nbs, an unspecific Nb (Nb Ctrl.) or phycoerythrin (PE)-labeled hOX40 mAb before (0 h, lower panel) and after (24 h, upper panel) stimulation with 5 µg/mL phytohaemagglutinin L (PHA-L); and

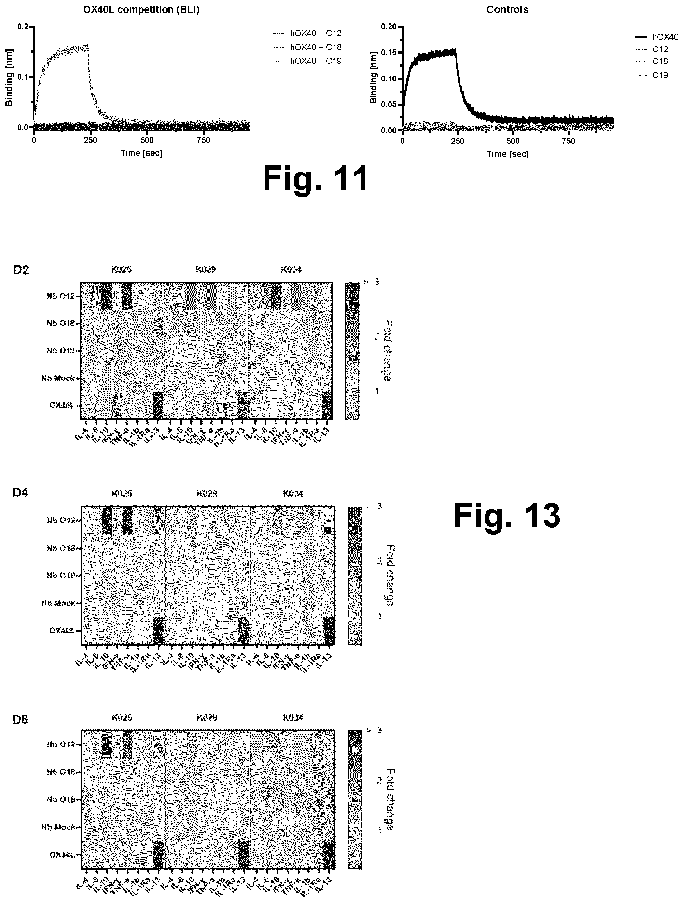

**Fig. 11** shows the results of an BLI-based OX40L competition assay: OX40L was immobilized to the sensor tips followed by addition of hOX40 either left untreated or preincubated with a 10-fold molar excess of Nb 012, 018 or O19. To exclude artefacts binding of each analyte to hOX40 was also tested alone (Controls);

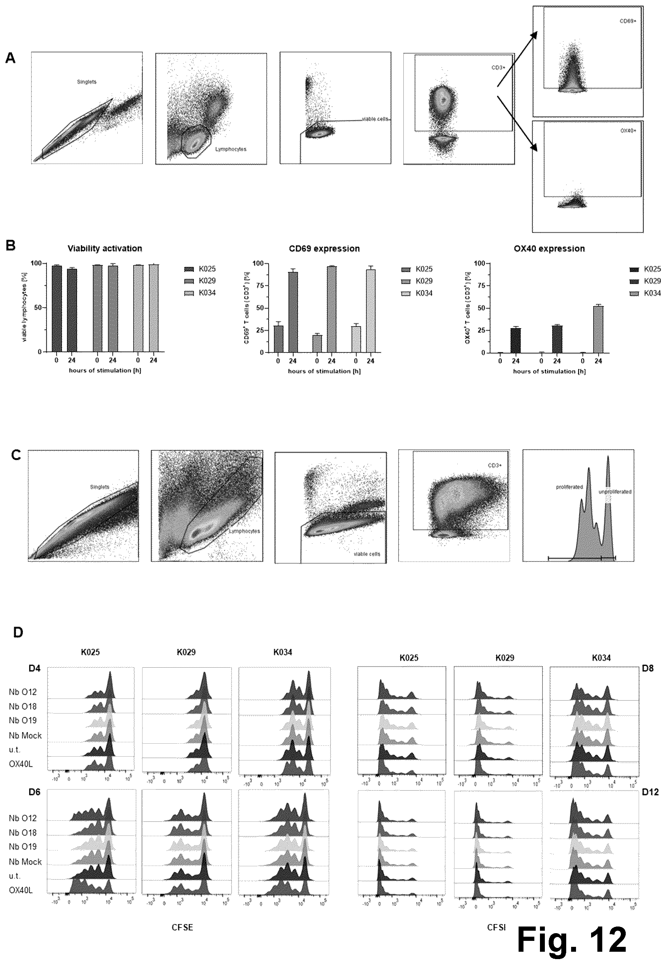

**Fig. 12** shows the results flow cytometry analysis of effects of hOX40-Nb binding: (**A**) Gating strategy for monitoring successful activation and hOX40 expression on hPBMCs upon PHA-L stimulation (exemplary shown for K034 at day 0). From left to right: single cells, lymphocytes, viable cells, CD3⁺ cells and CD69 expressing (upper gate) or OX40 expressing (lower gate) cells. (**B**) Viability (left), CD69 expression (mid) and OX40 expression (right) before and after stimulation for the three donors K025, K029 and K034. Data are presented as mean ± SD of three technical replicates (n = 3) (**C**) Gating strategy for T cell proliferation assay (exemplary shown for K034, 4 days after Nb treatment). From left to right: single cells, lymphocytes, viable cells, CD3⁺ cells and CFSE-low/negative cells. (**D**) Histogram overlay shows the number of divisions as CFSE labeling within CD3⁺ cells. Shown are all donors (K025, K029, K034) at days 4 (upper left panel), 6 (lower left panel), 8 (upper right panel) and 12 (lower right panel);
**Fig. 13** shows the results of experiments assessing the impact of hOX40-Nb treatment on cytokine release of hPBMCs: The impact of binding of hOX40-Nbs on the release of cytokine secretion was tested by treating hPBMCs of three donors (K25, K29, K34) with 0.5 µM hOX40-Nbs, unspecific Nb (Mock), OX40L or left untreated upon 24h stimulation with 5 µg/mL PHA-L, to induce OX40 expression. Cytokine release to the medium was monitored at day 2, 4 and 8 (D2, D4, D8) by a microsphere-based sandwich immunoassay (see table 3 below) and summarized in a heat map for each time point. Values are shown as fold change compared to the untreated control based on the mean of three technical replicates (n = 3); and
**Fig. 14** shows the sequence alignment of exemplary nanobodies of the invention, i.e., O7, 012, 018 and 019.

### DETAILED DESCRIPTION OF PREFERRED EMBODIMENTS

Within the present invention, a set of human OX40-specific nanobodies (in the following also: "Nbs") was generated, their binding properties on recombinant and cellular OX40 were examined, and their epitopes with sub-domain resolution were determined. Also, the nanobodies were developed to monitor the activation status of T cells by *in vivo* molecular imaging.

### 1. Materials and Methods

### Expression constructs

hOX40-encoding DNA (GenBank accession: NM_003327.3) was synthesized and cloned into Nhel and EcoRI site of pcDNA3.1(+) by GenScript Biotech. The vector backbone was changed by cutting with the restriction enzymes EcoRI and BstBl into a backbone comprising an internal ribosomal entry site (IRES) and genes for eGFP as reporter and Blasticidin S deaminase for antibiotics resistance from the expression construct as described previously (Traenkle B, et al., "Single-Domain Antibodies for Targeting, Detection, and In Vivo Imaging of Human CD4(+) Cells." Front Immunol. 2021;12:799910). For the generation of hOX40 domain deletion mutant expression constructs hOX40ΔD1 (aa 66-277), hOX40ΔD1-2 (aa 108-277), hOX40ΔD1-3 (aa 127-277) of UniProtKB P43489, respective fragments were amplified using the primers listed in table 1 below and genetically fused N-terminally to a SPOT-Tag (Virant D, et al., "A peptide tag-specific nanobody enables high-quality labeling for dSTORM imaging." Nat Commun. 2018;9(1):930). DNA encoding for murine OX40 (mOX40) was purchased from Sino Biological (Catalog Number MG50808-NM).

**Table 1: Primers used for fragment amplification**

| **Name** | **Sequence 5'- 3'** | **purpose** |
|---|---|---|
| CALL001 | GTCCTGGCTGCTCTTCTACAAGG (SEQ ID NO. 17) | |
| CALL002 | GGTACGTGCTGTTGAACTGTTCC (SEQ ID NO. 18) | |
| FR1-1 | CATGGCNSANGTGCAGCTGGTGGANTCNGGNGG (SEQ ID NO. 19) | |
| FR1-2 | CATGGCNSANGTGCAGCTGCAGGANTCNGGNGG (SEQ ID NO. 20) | |
| FR1-3 | CATGGCNSANGTGCAGCTGGTGGANAGYGGNGG (SEQ ID NO. 21) | |
| FR1-4 | CATGGCNSANGTGCAGCTGCAGGANAGYGGNGG (SEQ ID NO. 22) | |
| FR1-ext1 | GTAGGCCCAGCCGGCCATGGCNSANGTGCAGCTGGTGG (SEQ ID NO. 23) | Nb library generation |
| FR1-ext2 | GTAGGCCCAGCCGGCCATGGCNSANGTGCAGCTGCAGGA (SEQ ID NO. 24) | |
| FR4-1 | GATGCGGCCGCNGANGANACGGTGACCNGNRYNCC (SEQ ID NO. 25) | |
| FR4-2 | GATGCGGCCGCNGANGANACGGTGACCNGNGANCC (SEQ ID NO. 26) | |
| FR4-3 | GATGCGGCCGCNGANGANACGGTGACCNGRCTNCC (SEQ ID NO. 27) | |
| FR4-4 | GATGCGGCCGCRCTNGANACGGTGACCNGNRYNCC (SEQ ID NO. 28) | |
| FR4-5 | GATGCGGCCGCRCTNGANACGGTGACCNGNGANCC (SEQ ID NO. 29) | |
| FR4-6 | GATGCGGCCGCRCTNGANACGGTGACCNGRCTNCC (SEQ ID NO. 30) | |
| Ox40_2-4_1for | CAGTACATCAATGGGCGTGG (SEQ ID NO. 31) | |
| Ox40_2-4_1rev | GAGAGACTGCACGCACGCGATCCGGCATGGTGGCGCTAGCCAG (SEQ ID NO. 32) | hOX40 domain deletions |
| Ox40_2-4_2for | CGTGCGTGCAGTCTCTCACTGGAGCAGCCCGTGCGGGCCGG (SEQ ID NO. 33) | |
| Ox40_2-4_2rev | GCGGAATTCTCAGATCTTGGC (SEQ ID NO. 34) | |
| Ox402-4_3for | GGGAGACCCAAGCTGGCT (SEQ ID NO. 35) | |
| Ox40_P1-rev | GGCTGCTCCAGTGAGAGACTGCACGCACGC (SEQ ID NO. 36) | |
| Ox40_3-4-for | GCAGTCTCTCACTGGAGCAGCCGATGTCGAGCTGGCACCCAGCCCCTG(SEQ ID NO. 37) | |
| Ox40_4-for | GCAGTCTCTCACTGGAGCAGCCCTTGTCCTCCAGGGCACTTCTCCC (SEQ ID NO. 38) | |

### Stable cell line generation and culturing

U2OS cells (ATCC) were cultured according to standard protocols in Dulbecco's modified Eagle's medium (DMEM), supplemented with 10 % (v/v) FCS and 1 % (v/v) penicillin/streptomycin (all Thermo Fisher Scientific). Cultivation conditions were 37°C and 5 % CO₂ atmosphere in a humidified incubator and passaged using 0.05 % trypsin-EDTA (Thermo Fisher Scientific). Transfection of plasmid DNA was performed using Lipofectamine 2000 (Thermo Fisher Scientific) according to the manufacturer's protocol. To generate U2OS cells stably overexpressing hOX40 on their surface (U2OS-hOX40), 24 h after transfection, selection pressure by 5 µg/mL Blasticidine S (Sigma Aldrich) was applied for a period of two weeks. After single cell separation, monoclonal cells were analyzed for hOX40 expression using live-cell fluorescence microscopy.

### Animal immunization and hOX40 Nb library generation

The alpaca immunization was performed with the approval of the Government of Upper Bavaria (approval number: 55.2-1-54-2532.0-80-14). Two alpacas *(Vicugna pacos*) were immunized using the extracellular part of recombinant hOX40 (hOX40 AA Leu 29 - Ala 216) produced in human HEK293 cells (Acrobiosystems). During a period of 91 days, the animals were vaccinated six times at day 0, 21, 28, 35, 49, 87. The initial vaccination was performed with 560 µg followed by five booster injections each consisting of 280 µg hOX40 with Adjuvant F (Gebru). After the 91-day period, lymphocytes were isolated from -200 mL of blood performing Ficoll gradient centrifugation with lymphocyte separation medium (Carl Roth) and total RNA was extracted by NucleoSpin^{®} RNA II (Macherey&Nagel). The mRNA was subsequently transcribed into cDNA by the First Strand cDNA Synthesis Kit (GE Healthcare). The Nb repertoire was isolated as described in three subsequent PCR reactions using the following primer combinations: (1) CALL001 and CALL002, (2) forward primers FR1-1, FR1-2, FR1-3, FR1-4, and reverse primer CALL002, and (3) forward primers FR1-ext1 and FR1-ext2 and reverse primers FR4-1, FR4-2, FR4-3, FR4-4, FR4-5, and FR4-6 introducing Sfil and Notl restriction sites (see Traenkle B, *et al.,* above) (see table 1 above). This enables subcloning of Nb library into the pHEN4 phagemid vector (see Arbabi Ghahroudi M, et al., "Selection and identification of single domain antibody fragments from camel heavy-chain antibodies". FEBS Lett. 1997;414(3):521-6).

### Nb screening

The selection of hOX40-specific Nbs was performed by two consecutive rounds of phage display against immobilized recombinant antigen. For this purpose, electrocompetent TG1 *E. coli* bacteria were transformed with the hOX40-Nb library in pHEN4 and infected with M13K07 helper phages leading to the generation of hOX40 Nb-presenting phages. 1 × 10¹¹ phages were enriched by adsorption to streptavidin or neutravidin plates (Thermo Fisher Scientific) coated with hOX40 (5 µg/mL), biotinylated by Sulfo-NHS-LC-LC-Biotin (Thermo Fisher Scientific) in 5 molar excess at ambient temperature for 30 min and purified using Zeba^{™} Spin Desalting Columns 7 K MWCo 0.5 mL (Thermo Fisher Scientific) according to manufacturer's protocol. Antigen and phage blocking was performed with 5% milk in PBS-T during the first round or BSA in the second. Washing stringency was increased with each panning round and elution of bound phages was performed by 100 mM triethylamine pH 10 (TEA, Roth), followed by neutralization with 1 M Tris/HCl pH 7.4. For phage rescue, TG1 bacteria were infected with the eluted phages during their exponential growth phase, spread on selection plates for subsequent selection rounds and incubated at 37°C overnight. Enrichment of antigen-specific phages was monitored by counting colony forming units (CFUs).

### Whole-cell phage ELISA

Monoclonal phage ELISA was executed in a whole cell setting. Individual clones were picked, and phage production was induced as described above. For antigen presentation U2OS-hOX40 cells or wild type (wt) U2OS for background determination were seeded in a density of 2 × 10⁴ cells per well in 100 µL in 96-well cell culture plates (Corning) coated with poly-L-lysine (Sigma Aldrich) and grown overnight. The next day, 70 µL of phage supernatant was added to each cell type and incubated at 4°C for 3 h. Cells were washed 5 × with 5% FCS in PBS, followed by incubation with M13-HRP-labeled detection antibody (Progen, 1:2000 Dilution) for 1 h and washed again 3 × with 5% FCS in PBS. For the final detection, Onestep ultra TMB 32048 ELISA substrate (Thermo Fisher Scientific) was added to each well and incubated until the color changed. The reaction was stopped with 100 µL of 1 M H₂SO₄ and the signal was detected with the Pherastar plate reader at 450 nm. Phage ELISA-positive clones were defined by a 2-fold signal above U2OS-WT control cells.

### Protein expression and purification

For production, hOX40-Nbs were cloned into pHEN6 vector (see Arbabi Ghahroudi M, *et al.,* above), expressed in XL-1 and purified using immobilized metal affinity chromatography (IMAC) and size exclusion chromatography according to standard procedures as previously described (Traenkle B, *et al.,* above). Sortase A pentamutant (eSrtA) in pET29 was a gift from David Liu (Addgene plasmid # 75144) and expressed and purified as published (Chen I, et al., "A general strategy for the evolution of bond-forming enzymes using yeast display." Proc Natl Acad Sci USA. 2011;108(28):11399-404). The quality of all purified proteins was analyzed via standard SDS-PAGE under denaturizing and reducing conditions (5 min, 95°C in 2x SDS-sample buffer containing 100 mM Tris/HCl, pH 6.8; 2 % (w/v) SDS; 5 % (v/v) 2-mercaptoethanol, 10 % (v/v) glycerol, 0.02 % bromphenole blue). Proteins were visualized by InstantBlue Coomassie (Expedeon) staining or alternatively by immunoblotting transferring proteins to nitrocellulose membrane (GE Healthcare, Chicago, IL, USA) and detection using a primary anti-Penta-His antibody (Qiagen) and secondary donkey anti-mouse AF647 antibody (Invitrogen) on a Typhoon Trio scanner (GE-Healthcare, excitation 633 nm, emission filter settings 670 nm BP30).

### Biolayer interferometry (BLI)

The binding kinetics analysis of hOX40-Nbs was performed using the Octet RED96e system (Sartorius) applying manufacturer's recommendations. Therefore, 5 µg/mL of biotinylated hOX40-Nbs diluted in Octet buffer (PBS, 0.1 % BSA, 0.02 % Tween20) were immobilized on streptavidin coated biosensor tips (SA, Sartorius) for 30 s and unbound Nb was washed away. For the association step, a dilution series of hOX40 ranging from 0.2 nM - 320 nM were applied for 300 s followed by dissociation in Octet buffer for 720 s. Each concentration was normalized to a reference applying Octet buffer only for association. Data were analyzed using the Octet Data Analysis HT 12.0 software applying the 1:1 ligand-binding model and global fitting. For epitope binning 5 µg/mL of each Nb, except O7 due to its inappropriate dissociation behavior, was immobilized to SA tips and the association was performed with a premixture of OX40 (100 nM) and an excess of unbiotinylated second Nb (1000 nM). By analyzing the binding behavior of the premixture, conclusions about shared epitopes were drawn. To determine a potential competition of Nbs with the natural OX40 ligand OX40L, OX40L was biotinylated and 10 µg/mL were immobilized to the SA tips. Premixture of hOX40 with a ten-time molar excess of each Nb was applied for the association step.

### Live-cell immunofluorescence

U2OS-hOX40 cells, U2OS-WT or U2OS cells transiently expressing hOX40 domain deletion mutants or murine OX40 were plated at a density of 1 × 10⁴ cells per well in 100 µL of a µClear 96-well plate (Greiner Bio One, cat. #655090) and cultivated overnight at standard conditions. The next day, cells were stained with 2 µg/mL Hoechst33258 (Sigma Aldrich) for nuclear staining in live-cell visualization medium DMEMgfp-2 (Evrogen, cat. #MC102) supplemented with 10 % FCS for 30 min at 37°C. Afterwards 10 -1000 nM fluorescently labeled hOX40-Nbs, an unspecific Nb (Mock) or an OX40 antibody (positive control) were added and incubated for 30 min at 4°C. Staining solution was replaced by live-cell visualization medium DMEMgfp-2 with 10 % FCS and images were acquired with a MetaXpress Micro XL system (Molecular Devices) at 20 x magnification.

### Stability analysis

To assess the thermal stability of the Nbs, nanoscale differential scanning fluorimetry (nanoDSF) with the Prometheus NT.48 device (Nanotemper) was performed. Freshly thawed hOX40-Nbs were diluted to 0.25 mg/mL in PBS and measured at time point d0 and after an incubation period of ten days at 37°C (d10) using standard capillaries. A thermal gradient ramping from 20°C to 95°C was applied while measuring fluorescence ratios (F350/F330) and light scattering. Using PR. ThermControl v2.0.4 the melting (T_{M}) and aggregation (T_{Agg}) temperatures were determined.

### Fluorescent labeling of Nanobodies

For sortase A based coupling of 50 µM Nb were added to 250 µM sortase peptide (H-Gly-Gly-Gly-propyl-azide synthesized by Intavis AG) and 10 µM sortase A both dissolved in sortase buffer (50 mM Tris, and 150 mM NaCl, pH 7.4 at 4°C) and reaction was started by adding 10 mM CaCl₂ for 4 h at 4°C. To avoid reverse sortase reaction, sortase A and uncoupled Nb were removed by Ni-NTA affinity chromatography. The coupled Nbs were concentrated and residual peptide was depleted using Amicon ultra-centrifugal filters MWCO 3 kDa. Taking advantage of SPAAC (strain-promoted azide-alkyne cycloaddition) click chemistry reaction fluorescent labeling was performed by incubating azide-coupled Nbs with 2-fold molar excess of DBCO-AF647 (Jena Bioscience) for 2 h at room temperature. Subsequent dialysis (GeBAflex-tube, 6-8 kDa, Scienova) led to removal of excess of DBCO-AF647. As final polishing step a hydrophobic interaction chromatography (HIC, HiTrap Butyl-S FF, Cytiva) was performed to deplete unlabeled Nb. The final products were analyzed via SDS-PAGE and spectrophotometry.

### OX40 bioassay

The OX40 bioassay kit (Promega) was used to determine a potential agonistic activity of Nbs 012, 018 and 019 according to manufacturer's instructions. On the day before assay, thaw-and-use OX40 effector cells (Promega) were thawed and seeded into the inner 60 wells of two white 96 well assay plates cultured in assay buffer (RPMI1640 with 5% FBS) at standard conditions overnight. The next day OX40L and Nbs were serially diluted (OX40L: 50 - 0.0008 nM; Nbs: 2000-0.3 nM) in assay buffer, and 20 µL of the diluted recombinant proteins were added to the assay plate in triplicates. The assay plate was incubated at 37°C, 5 % CO₂ for 5 h. Afterwards, the assay plate was equilibrated to ambient temperature for 10 min. For detection of agonistic function, 75 µL of Bio-GloReagent was added to all wells. The assay plate was incubated at room temperature for 5 min, and luminescence was measured using a Tecan M2000 plate reader. The average relative luminescence unit (RLU) was calculated for each dilution. The average RLU data were plotted against the different concentrations of OX40L and hOX40-Nbs Nbs. To test antagonistic properties of the Nbs, the OX40 bioassay was transformed into a competition assay. For this purpose, the cells were pre-incubated with a serial dilution of hOX40-Nbs (0.13 µM to 0.002 nM) for one hour, followed by a 5 h incubation period with 0.12 µM OX40L.

### Peripheral blood mononuclear cells isolation and start of culture

Human peripheral blood mononuclear cells (hPBMCs) were isolated as described in (Traenkle , *et al.,* see above). In brief, Fresh blood, buffy coats, or mononuclear blood cell concentrates were obtained from healthy volunteers at the Department of Immunology or from the ZKT Tübingen gGmbH. Participants gave informed written consent and the studies were approved by the ethical review committee of the University of Tübingen, projects 156/2012B01 and 713/2018BO2. Blood products were diluted with PBS 1x (homemade from 10x stock solution, Lonza, Switzerland) and PBMCs were isolated by density gradient centrifugation with Biocoll separation solution (Biochrom, Germany). PBMCs were washed twice with PBS 1x, counted with a NC-250 cell counter (Chemometec, Denmark), and resuspended in heat-inactivated (h.i.) fetal bovine serum (Capricorn Scientific, Germany) containing 10% DMSO (Merck). For the experiments, cells were thawed in Iscove's Modified Dulbecco's Medium (IMDM + L-Glutamin + 25 mM HEPES; Thermo Fisher Scientific) supplemented with 2.5 % h.i. FBS (Thermo Fisher Scientific), 1 % P/S (Sigma-Aldrich), and 50 µM β-mercaptoethanol (β-ME; Merck), washed once, counted, and rested for 1 h at 37°C 5 % CO₂ in T cell media (TCM, IMDM + 1x P/S + 50 µM β-ME) supplemented with 1 µg/mL DNase I (Sigma-Aldrich). After resting, cells were washed once again, counted and used for subsequent analysis.

### Validation of hOX40-Nb binding to activated T cells

For the validation of Nb binding to activated T cells, hPBMCs were stained before and after stimulation for 24 h with 5 µg/mL PHA-L in TCM at 37°C 5 % CO₂. For flow cytometry analysis 2×10⁵ cells per staining condition in FACS buffer (PBS containing 0.02 % sodium azide, 2 mM EDTA, 5% hiFCFS) were used. Extracellular staining was performed with AF647-labeled hOX40-Nbs or unspecific (Mock) Nb (200 nM), phycoerythrin (PE)-labeled OX40 antibody (Ber-Act35, BioLegend), CD3 Ab APC/Cy7 (HIT3a, BioLegend), dead cell marker Zombie Violet (BioLegend) and isotype control Abs (BioLegend) each in pretested optimal concentrations by incubation for 30 min at 4°C. Cells were washed twice with FACS buffer and data were acquired on the same day using a LSRFortessa^{™} flow cytometer (Becton Dickinson) equipped with the DIVA Software (Becton Dickinson). Final data analysis was performed using the FIowJo10^{®} software (Becton Dickinson).

### T cell proliferation assay

The proliferation behavior of T cells was assessed using a carboxyfluorescein succinimidyl ester (CFSE) based approach. Up to 1×10⁸ cells were labeled with 2.5 µM CFSE (BioLegend) in 1 ml PBS for 20 min according to the manufacturer's protocol. The cells were washed twice in medium containing 10% h.i. FBS to stop CFSE labeling and stimulated for 24 h with 5 µg/mL PHA-L in TCM at 37°C 5 % CO₂ in a 48-well cell culture plate with 1.6-2.5×10⁶ cells/well. Induced OX40 expression was validated via flow cytometry. Subsequent to the stimulation, hPBMCs were treated with 0.5 µM of OX40 specific Nbs 012, 018 and 019, a control Nb, OX40L or left untreated and cultured at 37°C and 5 % CO₂. Concentrations were chosen in a clear excess than the expected concentration during clinical application. On days 3, 5 and 7, 2 ng/mL recombinant human IL-2 (R&D, USA) were added. One-third of the culture on day 4, one half of the culture on days 6 and 8, and the remaining cells on day 12 were harvested and counted. Cells from each condition were washed twice with FACS buffer (PBS containing 0.02% sodium azide, 2 mM EDTA, 5% hiFCFS). Extracellular staining was performed with CD3 Ab APC/Cy7 (HIT3a, BioLegend) and dead cell marker Zombie Violet (BioLegend) and isotype control Abs (BioLegend) each in pretested optimal concentrations by incubation for 30 min at 4°C. Cells were washed two times with FACS buffer and data were acquired on the same day using a LSRFortessaTM flow cytometer (Becton Dickinson) equipped with the DIVA Software (Becton Dickinson). Final data analysis was performed using the FlowJo100 software (Becton Dickinson). The percentage of proliferating T cells was determined by assessment of CFSE negative cells.

### Cytokine release assay

For cytokine release analysis, a set of in-house developed Luminex-based sandwich immunoassays was used. Supernatants from days 2, 4 and 8 of the proliferation assay were frozen at -80°C until cytokine measurements. Levels of IL-1b, IL-1Rα, IL-4, IL-6, IL-8, IL-10, IL-12p70, IL-13, granulocyte-macrophage colony-stimulating factor (GM-CSF), IFN-γ, macrophage chemotactic protein (MCP)-1, macrophage inflammatory protein (MIP)-1b, TNFα, and vascular endothelial growth factor (VEGF) were determined using a those immunoassays each consisting of commercially available capture and detection antibodies and calibrator proteins. All assays were thoroughly validated ahead of the study with respect to accuracy, precision, parallelism, robustness, specificity, and sensitivity (FDA, "Bioanalytical Method Validation: Guidance for Industry." US Department of Health and Human Services, Food and Drug Administration, Center for Drug Evaluation and Research, Center for Veterinary Medicine. 2018); EMEA, "Guideline on Bioanalytical Method Validation." European Medicines Agency; Committee for Medicinal Products for Human Use (CHMP). 2013). Samples were diluted at least 1:4 or higher. After incubation of the prediluted samples or calibrator protein with the capture coated microspheres, beads were washed and incubated with biotinylated detection antibodies. Streptavidin-phycoerythrin was added after an additional washing step for visualization. For control purposes, calibrators and quality control samples were included on each microtiter plate. All measurements were performed on a Luminex FlexMap^{®} 3D analyzer system using Luminex xPONENT^{®} 4.2 software (Luminex, USA). For data analysis, MasterPlex QT, version 5.0, was employed. Standard curve and quality control samples were evaluated according to internal criteria adapted to the Westgard Rules (Westgard JO, et al., "A multi-rule Shewhart chart for quality control in clinical chemistry." Clinical Chemistry. 1981;27(3):493-501) to ensure proper assay performance.

### hOX40-Nbs for in vivo optical imaging

For optical *in vivo* imaging, the Nb 018 was labeled with the fluorophore AlexaFluor647 (O18_{AF647}) by sortase-mediated attachment of an azide group followed by click-chemistry addition of DBCO-AF647. To establish hOX40⁺ expressing tumors, 5 × 10⁶ HT1080 cells stably expressing human OX40 (HT1080-hOX40) or 5 × 10⁶ wild type HT1080 (HT1080-WT) cells serving as negative control were resuspended in 50% Mat-rigel (BD) and 50% PBS and subcutaneously injected into the right upper flank of 7-week-old CD1 nude mice (Charles River Laboratories). When the tumors reached a size of 50 - 100 mm³, HT1080-hOX40 (n=3) or HT1080-WT (n=3) bearing mice were intravenously (i.v.) injected with 5 µg of Nb O18_{AF647} and noninvasively *in vivo* investigated by optical imaging (OI). For the *in vivo* measurements, the mice were anaesthetized with 1.5% isoflu-rane and the body temperature was kept constant at 37 °C using a heating mat. The mice were imaged over the course of 6 h and were sacrificed after the last imaging time point before tumors were explanted for ex *vivo* OI analysis. A bright field image and an image of the fluorescence signal (excitation 640 nm/ emission 680 nm) were recorded using an IVIS Spectrum OI System (PerkinElmer, Waltham, MA, USA). The fluorescence intensities were quantified by drawing regions of interest around the tumor borders and were expressed as average radiant efficiency (photons/s)/(µW/cm²) subtracted by the background fluorescence signal using the Living Image software 4.4 (Perkin Elmer). Statistic analyses were conducted with graph pad prism, Version 10. All mouse experiments were performed according to the German Animal Protection Law and were approved by the local authorities (Regierungspräsidium Tübingen).

### 2. Results

### Identification and characterization of hOX40 specific Nbs

For the generation of hOX40-specific Nbs, two alpacas (*Vicugna pacos*) were immunized with the extracellular domain of hOX40, which contains the amino acid residues from Leu29 to Ala216. A positive immune response in both animals was confirmed on day 63 by serum ELISA (data not shown). Starting from peripheral blood lymphocytes (PBLs), a Nb-phagemid library was established representing the VHH repertoire of both animals (size: ~3 × 10⁷ clones), from which hOX40-Nbs were enriched against recombinant hOX40 in two consecutive rounds of phage display. The selective binding of individual clones was tested in a whole-cell phage ELISA with U2OS cells stably expressing hOX40 (U2OS-hOX40). Subsequent sequencing revealed four unique hOX40-Nbs, namely O7, 012, 018 and O19, which exhibited highly diverse complementarity determining regions (CDRs) 3 (see Figure 1A, and table 2 below).

**Table 2: Amino acid sequences of identified hOX40-Nbs**

| **hOX40 Nb** | **Amino acid sequence** |
|---|---|
| **O7** | DVQLVESGGGLVQPGGSLRLSCAASGFTFGSYAMGWARQVPGKGLEWVSGIYSDGSTY YADSVKGRFTISRDNAKNTVYLQMNSLKPEDTAVYYCATWGAAAPYDYWGQGTQVTVSS (SEQ ID No. 16) |
| **O12** | DVQLQESGGGLVQPGGSLRLSCAASGFTLDNYAIGWFRQAPGKEREGVSCISSSGESTN YADSVKGRFTISRDNAKNTVYLQTNSLKPEDTAVYYCAAVDDIGTTVQFMCNMGPYEYDY WGQGSPVTVSS (SEQ ID No. 10) |
| **O18** | QVQLVESGGGLVQPGGSLRLSCAASGFTLEDYVIGWFRQAPGKEREEVSCISGSGGIRNV ADSMEGRATISRDNAKNTVYLQMNSLKPEDTAVYYCAAGFETSYSYSYSYYCGVHEYDYWG QGTLVTVSS (SEQ ID No. 11) |
| **O19** | QVQLQESGGGLVQPGGSLRLSCAGSTDTFSMSAAVSWARQAPGKGLEWVSSILSDGSTY YADSVRGRFTISRDNANNTGSLQMNNLKVEDTAVYYCTNLRGRLWSNHKDDYGGQGSRV TVSS (SEQ ID No. 12) |

All selected Nbs were expressed in *Escherichia coli (E. coli)* and purified by immobilized metal ion affinity chromatography (IMAC) followed by size exclusion chromatography (SEC), yielding high purity binding molecules (Figure 1B). To initially assess their binding affinities, biolayer interferometry (BLI) was performed and K_{D} values in the pico- to low nanomolar range (0.2 - 3.4 nM) were determined, while O7 showed a substantially weaker affinity as reflected by a K_{D} of ~ 140 nM (Figure 1C, and Fig. 7A). In addition, the folding stability of the selected candidates was determined using differential scanning fluorimetry (nanoDSF). All Nbs showed high thermal stabilities with melting temperatures (T_{M}) between 50°C and 74°C without aggregation. Notably, this was not affected by an accelerated aging period of 10 days at 37°C (see Figure 1D and 7B).

For the fluorescent functionalization of hOX40-Nbs, a sortase-based approach was applied to selectively attach an azide-group to the C-termini of the Nbs, which served as chemical handle for the addition of a AlexaFluor647(AF647)-conjugated diben-zocyclooctyne (DBCO-AF647) group utilizing click chemistry (Traenkle B, *et al.,* see above). As a result, Nbs comprising a C-terminal fluorophore with a defined labeling ratio of 1:1 were obtained. The fluorescent Nbs were used to determine corresponding EC₅₀ values on U2OS-hOX40 cells by flow cytometry. In accordance with the BLI-determined affinities, a strong functional binding for 012 and 018 with EC₅₀ values in the subnanomo-lar range (~ 0.1 nM for 012; ~ 0.3 nM for 018) was determined, whereas O7 and 019 displayed slightly weaker cellular affinities (Figure 2A, and Fig. 8A). To further confirm specific binding of the selected Nbs to hOX40 localized at the plasma membrane of mammalian cells, the fluorescent Nbs were used for live cell staining of U2OS-hOX40 cells in comparison to wild-type U2OS (U2OS-WT) cells. The images displayed intense signals localized at the cellular surface for all tested binders, which was comparable to the staining with an established hOX40 mAb, while none of the tested Nbs showed non-specific binding to U2OS-WT cells (Figure 2B). In addition, this approach was used to test a potential cross-reactivity of the Nbs to murine OX40 (mOX40) and performed live cell imaging on U2OS cells transiently expressing the mOX40. Only O7 bound to mOX40, while all other candidates showed no staining of mOX40 expressing U2OS cells (see Fig. 8B). In summary, four hOX40-Nbs were identified that bind recombinant as well as cell-resident hOX40. With respect to 012, 018 and 019, high-affinity binders with K_{D} values in the pico to low nanomolar range were selected that exhibited strong specific binding to membrane-exposed hOX40. Notably, only O7 was less affine to hOX40 but showed additional cross-reactivity towards mOX40.

### Characterization of binding epitopes on hOX40

To localize the binding sites of the selected hOX40-Nbs within the natively folded hOX40, cellular expression constructs were generated comprising domain-deletion mutants of hOX40 domains 1-3, which were transiently expressed in U2OS cells. Nb binding to truncated versions of hOX40 was visualized by immunofluorescence imaging of live cells. An anti-hOX40 mAb directed against domain 4 was used as a positive control (Figure 3A). Based on these results, binding of O7 and 019 was allocated to domain 3 as well as 012 and 018 to domain 1 of hOX40 (Figure 3B). To examine a potential combinatorial binding of the different hOX40-Nbs, epitope binning analysis was performed by BLI (Figure 3C). As expected from the domain mapping, 012 and O18, both targeting domain 1, simultaneously bound hOX40 in complex with O7 or 019, each targeting domain 3. However, only weak combinatorial binding was observed for Nbs addressing the same domain, suggesting that 012 and 018, as well as O7 and 019 address identical or at least overlapping epitopes (Figure 3D and Fig. 9).

### hOX40-Nbs bind to activated human T lymphocytes

Having demonstrated that all selected Nbs recognize recombinant and exogenously overexpressed cellular hOX40, their specificity for binding to endogenous hOX40 on activated T cells was investigated. Therefore, human peripheral blood mononuclear cells (hPBMCs) from three healthy donors (K025, K029 and K034) were either left untreated or incubated for 24 h with phytohaemagglutinin L (PHA-L) as a non-antigenic stimulus to induce expression of OX40 (Figure 4A). Subsequently, hPBMCs were double-stained with fluorescently labeled hOX40-Nbs or a phycoerythrin (PE)-labeled anti-hOX40 mAb in combination with a T cell-specific anti-CD3 mAb, and the percentage of double-positive T cells (CD3⁺OX40⁺) was analyzed by flow cytometry (Figure 4C, gating: Fig. 10). All hOX40-Nbs except O7 bound specifically to T cells upon PHA-L-mediated activation, comparable to the anti-hOX40 mAb. Notably, no binding prior to stimulation was observed (Figure 4B,C). These data indicated that 012, 018 and 019 bound endogenous hOX40 exclusively on activated T cells.

### Agonistic and antagonistic effects of Nbs on OX40 signaling

Targeting OX40, can trigger strong immune responses. Therefore, it was investigated whether binding of the Nbs exerts agonistic or antagonistic effects on OX40-mediated signaling by using a genetically engineered Jurkat T cell-based bioassay. These effector cells express hOX40 and contain a luciferase reporter driven by a response element downstream of the OX40 signaling axis. Non-stimulated OX40 effector cells exhibited a weak luminescent signal, which was not further enhanced by addition of increasing concentrations of Nbs 012, 018, and 019. However, when using OX40L as positive control, a strong concentration-dependent induction of NF-kB promotor activity was observed, reflected by an increasing luminescence signal, as expected (Figure 5A). Next, this assay was used to analyze a possible competition between OX40L and Nbs for hOX40 binding. Therefore, the OX40 effector cells were preincubated with serial dilutions of Nbs ranging from 0.13 µM to 0.002 nM before adding OX40L at the saturation concentration of 0.12 µM. In this setting, a reduction in luminescence in the presence of Nb 012 and, to a minor extent, for O18, was observed, whereas pre-incubation of 019 did not have any effect on OX40L-mediated induction of OX40 signaling (Figure 5B). These results were consistent with a BLI-based competition assay in which the binding of OX40L to recombinant hOX40 in the presence or absence of Nbs (see Fig. 11) was monitored. From these findings, it was concluded that none of the Nbs augment OX40-mediated signaling. However, 012 and, to a minor degree, 018, which both target domain 1 of hOX40, competed with the binding of the natural ligand OX40L and could therefore potentially exert an antagonistic effect.

### Impact of hOX40-Nbs on proliferation and cytokine release of immune cells

To further explore possible effects of hOX40-Nb binding on T cells, next, its influence on proliferation and cytokine release in hPBMCs was investigated. Therefore, hPBMCs from three donors (K025, K029 and K034) were labeled with carboxyfluorescein succinimidyl ester (CFSE) followed by induction of hOX40 expression by PHA-L stimulation for 24 h. After confirming a successful activation and hOX40 expression by flow cytometry (see fig. 12 A, B), hPBMCs were either left untreated or incubated with Nbs 012, 018, and 019 or PEP-Nb (Nb Mock) as a negative control (each 0.5 µM). For targeted hOX40 stimulation 0.5 µM OX40L was used (Figure 5C). Cell proliferation was monitored on days 4, 6, 8 and 12 by flow cytometry (Figure 5D). The results revealed similar proliferation profiles in the hPBMC samples from the same donor upon Nb treatment compared to the untreated samples, while treatment with OX40L induced a substantial increase in proliferation (Figure 5D; and Fig. 12 C, D). In addition, effects of hOX40-Nbs on the release of cytokines were investigated. Therefore, the concentration of a panel of pro- and anti-inflammatory cytokines was determined (see Table 3 below) in the supernatant of samples collected on days 2, 4, and 8 using a previously reported microsphere-based sandwich immunoassay (Traenkle B, *et al.,* above).

**Table 3: Cytokines analyzed in this study**

| **Cytokine** | **indicative for** |
|---|---|
| Interleukin 1b (IL-1b) | proinflammatory |
| Interleukin 1 receptor antagonist (IL-1Ra) | antiinflammatory |
| Interleukin 4 (IL-4) | antiinflammatory |
| Interleukin 6 (IL-6) | pro-/antiinflammatory |
| Interleukin 10 (IL-10) | antiinflammatory |
| Interleukin 13 (IL-13) | antiinflammatory |
| Interferon γ (IFN-γ) | proinflammatory |
| Tumor necrosis factor α (TNF-α) | proinflammatory |

While Nb 018 and 019 showed only minor effects on cytokine release compared to the untreated control or samples incubated with a non-specific Nb, a significant increase of the NFκ-driven cytokines TNF and IL-6 and of the Th2 cytokines IL-4, and IL-10 upon treatment with O12 was observed. Interestingly, the cytokine release of O12-treated samples differed from samples treated with OX40L, in which only elevated levels of IL-13 were observed (Figure 5E, and Fig. 13).

### hOX40-Nb for in vivo imaging

For *in vivo* optical imaging (OI), the fluorescently-labeled Nb 018 (O18_{AF647}) was chosen as a non-modulating candidate, because it showed the strongest binding to cellular exposed hOX40. CD1 nude mice with subcutaneous HT1080-hOX40 or HT1080-WT tumors were intravenously (*i.v*.) injected with 5 µg of O18_{AF647} and non-invasively *in vivo* investigated by OI over 6 h. The signal intensity (SI) of O18_{AF647} in both the HT1080-hOX40 and the HT1080-WT tumors peaked within 5 min after injection and decreased to -50% after 60 min. At all measured time points, the O18_{AF647} related SI within the TME increased in the HT1080-hOX40 tumors compared to HT1080-WT tumors and remained stable between 3 h and 6 h post injection. In sharp contrast, the OX40-related SI continuously decreased in the non-OX40 expressing WT tumors. Six hours after injection, O18_{AF647} showed the greatest difference in the SI between both groups, revealing a ∼7-fold higher SI within the TME of HT1080-hOX40 compared to the SI in non-OX40 expressing HT1080-WT tumors (Figure 6A, B). Finally, mice were sacrificed, and the presence of O18_{AF647} within the explanted tumors was analyzed by *ex vivo* OI. Consistent with the *in vivo* data, HT1080-hOX40 tumors exhibited a significantly higher uptake (SI) within the TME when compared to HT1080-WT control tumors, indicating a specific binding of the Nb 018 to its target antigen and a good signal-to-background ratio for this Nb-derived immunoprobe (Figure 6C). These findings demonstrated a specific binding for O18_{AF647}, highlighting its potential as a promising tool for noninvasive monitoring of OX40 expression in immune diagnostics.

### 3. Summarizing Discussion

With the above results and experiments it could be shown that the nanobodies of the invention represent suitable tools for specifically recognizing and binding hOX40, offering the possibility to use the nanobodies for monitoring, diagnosing and prediction applications, e.g., in the field of immunotherapies.

E.g., via the nanobodies of the invention, and their binding to hOX40-expressing activated T cells, the outcome of tumor therapies can be monitored, since on tumor infiltrating T cells, expression of hOX40 correlates with a beneficial outcome and overall survival of patients with solid tumors.

Also, the nanobody of the invention is suitable in the diagnosis or prediction of immune-mediated diseases (IMIDs), where an increased expression of hOX40 can be correlated with disease activity and severity of these diseases.

The presented nanobodies have a high binding affinity and long-term stability. None of the hOX40-Nbs shown herein induced OX40 signaling or affected T cell proliferation.

With the nanobodies of the invention, it could also be shown that a site-directed functionalization employing C-terminal sortagging and DBCO-mediated click chemical conjugation to AF647 did not affect their binding properties. Further, the nanobodies of the invention were successfully used in optical imaging in a mouse xenograft model, where a rapid accumulation of the labeled nanobodies to HT1080-hOX40 tumors and sustained binding over a prolonged period of time could be shown, additionally proving the a high *in vivo* binding functionality with a low off-rate.

In summary, the specific detection of activated T cells can support therapy monitoring and patient selection for personalized immunotherapies such as immune checkpoint inhibitor therapies, where a high number of activated T cells within the TME is beneficial. Furthermore, the nanobodies of the invention enable, e.g., the evaluation of responses to cancer vaccination or cytokine therapy and provides information on the activation status of CAR-T cells.

Due to the high binding functionality and unique properties in terms of tissue penetration and improved signal-to-noise ratio without unwanted modulation of OX40 signaling, the hOX40-Nbs of the invention will facilitate the visualization of even small numbers of OX40-activated T cells. Because of their fast pharmacokinetics, the nanobodies of the invention enable earlier imaging timepoints, and, therefore, the usage of shorter-lived isotopes, e.g., ¹⁸F, thereby reducing patients' radiation exposure and allowing more longitudinal imaging to assess dynamic changes in the T cell composition within the TME, IME and the lymphatic organs.

Summarizing, one of the most significant advancements facilitated by these novel tools and probes is in the realm of personalized immunotherapy. Personalized immunotherapy represents a transformative approach in treating cancer and immune-mediated diseases, focusing on tailoring treatments to the individual patient's unique immune profile. By enabling real-time monitoring of activated T cells, this invention allows for the dynamic assessment of a patient's immune response to specific therapies.

## Claims

1. A nanobody which specifically binds to human OX40 (hOX40), wherein the nanobody does not have an agonistic effect on hOX40, the nanobody comprising
a) the amino acid sequences (i) SGFTLDNY (SEQ ID NO: 1) as CDR1, (ii) ISSSGESTNYAD (SEQ ID NO: 2) as CDR2 and (iii) VDDIGTTVQFMCNMGPYEYD (SEQ ID NO: 3) as CDR3; or
b) the amino acid sequences (i) SGFTLEDY (SEQ ID NO: 4) as CDR1, (ii) ISGSGGIRNVAD (SEQ ID NO: 5) as CDR2 and (iii) GFETSYSYSYYCGVHEYD (SEQ ID NO: 6) as CDR3; or
c) the amino acid sequences (i) STDTFSMSA (SEQ ID NO: 7) as CDR1, (ii) ILSDGSTYYAD (SEQ ID NO: 8) as CDR2 and (iii) LRGRLWSNHKDD (SEQ ID NO: 9) as CDR3; or
d) the amino acid sequences (i) SGFTFGSY (SEQ ID No. 13) as CDR1, (ii) IYSDGSTYYAD (SEQ ID No. 14) as CDR2 and (iii) WGAAAPYD (SEQ ID No. 15) as CDR3; or
e) amino acid sequences that have at least 85% sequence homology with the amino acid sequences as defined in a), b), c), or d); or
or a functionally conservative variant of the nanobody as defined in any of a), b), c), or d), comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 13, SEQ ID No. 14, and SEQ ID No. 15.

2. The nanobody of claim 1, wherein the nanobody binds to i) domain D1, or to ii) domain D3, of the extracellular region of hOX40.

3. The nanobody of claim 1 or 2, wherein the nanobody binds to recombinant and exogenously expressed hOX40, and/or to endogenous hOX40 on activated T cells.

4. The nanobody of any of claims 1 to 3, wherein the nanobody is an hOX40-OX40L-interaction-blocking hOX40-nanobody, or an inert hOX40 nanobody.

5. The nanobody of any of claims 1 to 4, comprising four framework regions (FR1 to FR4) and three complementarity determining regions (CDR1 to CDR3), the three complementarity determining regions consisting of
(i) one of the amino acid sequences a), b), c), d), or of
(ii) an amino acid sequence that has at least 85% sequence homology with one of the amino acid sequences a), b), c), d) or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 13, SEQ ID No. 14, and SEQ ID No. 15;
preferably, wherein the nanobody comprises or consists of an amino acid sequence selected from the group consisting of the amino acid sequences SEQ ID NO. 10, SEQ ID NO. 11, or SEQ ID NO. 12; or wherein the nanobody comprises or consists of an amino acid sequence that has at least 85% sequence homology with one of the amino acid sequences SEQ ID NO. 10, SEQ ID NO. 11, SEQ ID No. 12, or SEQ ID No. 16.

6. The nanobody of any of claims 1 to 5, wherein the nanobody is directly or indirectly associated with at least one of a detectable label.

7. The nanobody of claim 6, wherein the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

8. A nucleic acid comprising or consisting of a nucleic acid sequence coding for the nanobody as claimed in any of claims 1 to 6, optionally linked to another nucleic sequence.

9. The nanobody of any of claims 1 to 7, or the nucleic acid of claim 8, for use in diagnostic or prognostic methods.

10. The nanobody of any of claims 1 to 7, or 9, for use as contrast agent in non-invasive medical imaging *in vivo*, preferably for identifying the presence, absence, and/or amount of hOX40-expressing cells, preferably of hOX40-expressing activated T cells, preferably of hOX40-expressing activated T cells within the tumor microenvironment (TME), the inflammatory microenvironment (IME) in immune-mediated diseases (IMIDs) and the lymphatic organs.

11. The nanobody for use of claim 10, wherein the nanobody is used for monitoring of hOX40 expression in cancer diagnostics, diagnostics of immune mediated diseases, and/or evaluation of immunotherapy.

12. A biparatopic, bivalent or trivalent construct comprising at least one nanobody as claimed in any of claims 1 to 7, or a combination thereof.

13. A method for determining and/or imaging and/or monitoring the distribution, presence and/or amount of hOX40-expressing cells in a biological sample suspected of comprising hOX40-expressing cells, the method comprising: contacting the biological sample or tissue with the nanobody as claimed in any of claims 1 to 7, or with a construct of claim 12, and determining and/or imaging and/or monitoring the distribution, presence and/or amount of hOX40-expressing cells in the biological sample or tissue.

14. The method of claim 13, wherein the method comprises the steps of:
a) combining said sample with at least one of the nanobodies of any of claims 1 to 7, or with at least one construct of claim 12, and optionally with at least one second compound conjugated with a detectable label, the second compound binding to another marker of the hOX40 expressing cells,
b) measuring, via the detectable label, imaging signals from said nanobody that has bound to hOX40 expressing cells in said sample, and, if a second compound has additionally been used in step a), from said second compound that has bound to another marker; and
c) imaging, determining and/or monitoring the presence and/or amount and/or activity of hOX40 expressing cells in said sample via the imaging signals.

15. A method for diagnosing and/or monitoring the immune status and/or susceptibility to immunotherapies of a patient, wherein at least one nanobody as claimed in any of claims 1 to 7, or at least one construct as claimed in claim 12, is used to assess and/or monitor hOX40 expressing cells in a biological sample of the patient, wherein the nanobody is used alone, or in combination with a second compound binding to a T-cell specific marker, wherein the T cell-specific marker is selected from at least one of CD2, CD3, CD4, CD7, CD8, CD27, CD28, CD127, HLA-DR, CD38, CD69, CCR4, CCR5, CCR6, CCR7, CTLA-4, LAG3, TIM-3, ICOS, CXCR4, PD-1, PD-L1, PD-L2, CD40L (synonym CD154), CD122, CD137, GITR, CD25, CD278, SIGLEC-7, SIGLEC-9, BTLA (synonym: CD272), TIGIT, VISTA, B7-H4 (synonym: VTCN1), CD276 (synonym: B7-H3), A2AR, CEACAM1, LAIR-3, HVEM, CD160, CD200, CD200R.

16. The method of claim 15, wherein the nanobody is detectably labeled, preferably radiolabeled, and wherein in the determining and/or imaging and/or monitoring step the presence, or absence, or distribution, or amount of the label is detected.

17. A cell line producing a nanobody of any of claims 1 to 7.

18. Use of at least one of the nanobodies of any of claims 1 to 7, or at least one construct of claim 12, as a diagnostic agent, preferably wherein the diagnostic agent is used in a diagnosis which is selected from diagnostic classification of a disease-associated immune status of a patient, and susceptibility to immunotherapies of a patient.

19. A kit comprising at least one nanobody of any of claims 1 to 7, the nucleic acid as claimed in claim 8, or a cell of a cell line of claim 17; and a detectable marker.
